# EUROPEAN PATENT APPLICATION

(11) **EP 2 258 723 A1**
(43) Date of publication of application: **08.12.2010**
(21) Application number: 09007319.8
(22) Date of filing: 02.06.2009
(51) Int. Cl.: C07K 16/28, C07K 16/24, A61K 39/395

(54) **Treatment of oncostatin M receptor ß mediated heart failure**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner

(57) **Abstract**

The present invention relates to an inhibitor of the oncostatin M receptor β or an inhibitor of an activator of the oncostatin M receptor β for use in the treatment and/or prevention of heart failure. The present invention also relates to a method of treating and/or preventing heart failure comprising administering a pharmaceutically effective amount of an inhibitor of the oncostatin M receptor β or an inhibitor of an activator of the oncostatin M receptor β to a subject in need thereof. Further, the present invention also relates to methods of identifying a compound suitable as a lead compound and/or as a medicament for the treatment and/or prevention of heart failure.

SEQUENCE LISTING

<110> Max-Planck-Gesellschaft zur Förderung der Wissenschaften e.V.

<120> Treatment of oncostatin M receptor ß mediated heart failure

<130> R1114 EP

<160> 4

<170> PatentIn version 3.4

<210> 1
<211> 979
<212> PRT
<213> homo sapiens

<400> 1

<210>  2
<211>  4171
<212>  DNA
<213>  homo sapiens

<400> 2
gggccgcctc tgcacgtccg ccccggagcc cgcacccgcg ccccacgcgc cgccgaggac 60
tcggcccggc tcgtggagcc cttcgcccgc ggcgtgagta cccccgaccc gcccgtcccc 120
gctctgctcg cgccctgccg ctgcgccgcc ctcggtggct tttccgacgg gcgagccccg 180
tgctgtgcgg gaaagaatcc gacaacttcg cagcccatcc cggctggacg cgaccgggag 240
tgcagcagcc cgttcccctc ctcggtgccg cctctgccca gcgtttgctt ggctgggcta 300
ccacctgcgc tcggacggcg ctcggagggt cctcgccccc ggcctgccta cctgaaaacc 360
agaactgatg gctctatttg cagtctttca gacaacattc ttcttaacat tgctgtcctt 420
gaggacttac cagagtgaag tcttggctga acgtttacca ttgactcctg tatcacttaa 480
agtttccacc aattctacgc gtcagagttt gcacttacaa tggactgtcc acaaccttcc 540
ttatcatcag gaattgaaaa tggtatttca gatccagatc agtaggattg aaacatccaa 600
tgtcatctgg gtggggaatt acagcaccac tgtgaagtgg aaccaggttc tgcattggag 660
ctgggaatct gagctccctt tggaatgtgc cacacacttt gtaagaataa agagtttggt 720
ggacgatgcc aagttccctg agccaaattt ctggagcaac tggagttcct gggaggaagt 780
cagtgtacaa gattctactg gacaggatat attgttcgtt ttccctaaag ataagctggt 840
ggaagaaggc accaatgtta ccatttgtta cgtttctagg aacattcaaa ataatgtatc 900
ctgttatttg gaagggaaac agattcatgg agaacaactt gatccacatg taactgcatt 960
caacttgaat agtgtgcctt tcattaggaa taaagggaca aatatctatt gtgaggcaag 1020
tcaaggaaat gtcagtgaag gcatgaaagg catcgttctt tttgtctcaa aagtacttga 1080
ggagcccaag gacttttctt gtgaaaccga ggacttcaag actttgcact gtacttggga 1140
tcctgggacg gacactgcct tggggtggtc taaacaacct tcccaaagct acactttatt 1200
tgaatcattt tctggggaaa agaaactttg tacacacaaa aactggtgta attggcaaat 1260
aactcaagac tcacaagaaa cctataactt cacactcata gctgaaaatt acttaaggaa 1320
gagaagtgtc aatatccttt ttaacctgac tcatcgagtt tatttaatga atccttttag 1380
tgtcaacttt gaaaatgtaa atgccacaaa tgccatcatg acctggaagg tgcactccat 1440
aaggaataat ttcacatatt tgtgtcagat tgaactccat ggtgaaggaa aaatgatgca 1500
atacaatgtt tccatcaagg tgaacggtga gtacttctta agtgaactgg aacctgccac 1560
agagtacatg gcgcgagtac ggtgtgctga tgccagccac ttctggaaat ggagtgaatg 1620
gagtggtcag aacttcacca cacttgaagc tgctccctca gaggcccctg atgtctggag 1680
aattgtgagc ttggagccag gaaatcatac tgtgacctta ttctggaagc cattatcaaa 1740
actgcatgcc aatggaaaga tcctgttcta taatgtagtt gtagaaaacc tagacaaacc 1800
atccagttca gagctccatt ccattccagc accagccaac agcacaaaac taatccttga 1860
caggtgttcc taccaaatct gcgtcatagc caacaacagt gtgggtgctt ctcctgcttc 1920
tgtaatagtc atctctgcag accccgaaaa caaagaggtt gaggaagaaa gaattgcagg 1980
cacagagggt ggattctctc tgtcttggaa accccaacct ggagatgtta taggctatgt 2040

tgtggactgg tgtgaccata cccaggatgt gctcggtgat ttccagtgga agaatgtagg 2100
tcccaatacc acaagcacag tcattagcac agatgctttt aggccaggag ttcgatatga 2160
cttcagaatt tatgggttat ctacaaaaag gattgcttgt ttattagaga aaaaaacagg 2220
atactctcag gaacttgctc cttcagacaa ccctcacgtg ctggtggata cattgacatc 2280
ccactccttc actctgagtt ggaaagatta ctctactgaa tctcaacctg gttttataca 2340
agggtaccat gtctatctga aatccaaggc gaggcagtgc cacccacgat ttgaaaaggc 2400
agttctttca gatggttcag aatgttgcaa atacaaaatt gacaacccgg aagaaaaggc 2460
attgattgtg gacaacctaa agccagaatc cttctatgag tttttcatca ctccattcac 2520
tagtgctggt gaaggcccca gtgctacgtt cacgaaggtc acgactccgg atgaacactc 2580
ctcgatgctg attcatatcc tactgcccat ggttttctgc gtcttgctca tcatggtcat 2640
gtgctacttg aaaagtcagt ggatcaagga gacctgttat cctgacatcc ctgaccctta 2700
caagagcagc atcctgtcat taataaaatt caaggagaac cctcacctaa taataatgaa 2760
tgtcagtgac tgtatcccag atgctattga agttgtaagc aagccagaag ggacaaagat 2820
acagttccta ggcactagga agtcactcac agaaaccgag ttgactaagc ctaactacct 2880
ttatctcctt ccaacagaaa agaatcactc tggccctggc ccctgcatct gttttgagaa 2940
cttgacctat aaccaggcag cttctgactc tggctcttgt ggccatgttc cagtatcccc 3000
aaaagcccca agtatgctgg gactaatgac ctcacctgaa aatgtactaa aggcactaga 3060
aaaaaactac atgaactccc tgggagaaat cccagctgga gaaacaagtt tgaattatgt 3120
gtcccagttg gcttcaccca tgtttggaga caaggacagt ctcccaacaa acccagtaga 3180
ggcaccacac tgttcagagt ataaaatgca aatggcagtc tccctgcgtc ttgccttgcc 3240
tcccccgacc gagaatagca gcctctcctc aattaccctt ttagatccag gtgaacacta 3300
ctgctaacca gcatgccgat ttcatacctt atgctacaca gacattaaga agagcagagc 3360
tggcaccctg tcatcaccag tggccttggt ccttaatccc agtacaattt gcaggtctgg 3420
tttatataag accactacag tctggctagg ttaaaggcca gaggctatgg aacttaacac 3480
tccccattgg agcaagcttg ccctagagac ggcaggatca tgggagcatg cttaccttct 3540
gctgtttgtt ccaggctcac ctttagaaca ggagacttga gcttgaccta aggatatgca 3600
ttaaccactc tacagactcc cactcagtac tgtacagggt ggctgtggtc ctagaagttc 3660
agtttttact gaggaaatat ttccattaac agcaattatt atattgaagg ctttaataaa 3720
ggccacagga gacattacta tagcatagat tgtcaaatgt aaatttactg agcgtgtttt 3780
ataaaaaact cacaggtgtt tgaggccaaa acagatttta gacttacctt gaacggataa 3840
gaatctatag ttcactgaca cagtaaaatt aactctgtgg gtgggggcgg ggggcatagc 3900
tctaatctaa tatataaaat gtgtgatgaa tcaacaagat ttccacaatt cttctgtcaa 3960
gcttactaca gtgaaagaat gggattggca agtaacttct gacttactgt cagttgtact 4020
tctgctccat agacatcagt attctgccat catttttgat gactacctca gaacataaaa 4080

aggaacgtat atcacataat tccagtcaca gtttttggtt cctcttttct ttcaagaact 4140

atatataaat gacctgtttt cacgcggccg c 4171

<210> 3
<211> 252
<212> PRT
<213> homo sapiens

<400> 3

Met Gly Val Leu Leu Thr Gln Arg Thr Leu Leu Ser Leu Val Leu Ala
1               5                   10                  15

Leu Leu Phe Pro Ser Met Ala Ser Met Ala Ala Ile Gly Ser Cys Ser
            20                  25                  30

Lys Glu Tyr Arg Val Leu Leu Gly Gln Leu Gln Lys Gln Thr Asp Leu
        35                  40                  45

Met Gln Asp Thr Ser Arg Leu Leu Asp Pro Tyr Ile Arg Ile Gln Gly
    50                  55                  60

Leu Asp Val Pro Lys Leu Arg Glu His Cys Arg Glu Arg Pro Gly Ala
65                  70                  75                  80

Phe Pro Ser Glu Glu Thr Leu Arg Gly Leu Gly Arg Arg Gly Phe Leu
                85                  90                  95

Gln Thr Leu Asn Ala Thr Leu Gly Cys Val Leu His Arg Leu Ala Asp
            100                 105                 110

Leu Glu Gln Arg Leu Pro Lys Ala Gln Asp Leu Glu Arg Ser Gly Leu
        115                 120                 125

Asn Ile Glu Asp Leu Glu Lys Leu Gln Met Ala Arg Pro Asn Ile Leu
    130                 135                 140

Gly Leu Arg Asn Asn Ile Tyr Cys Met Ala Gln Leu Leu Asp Asn Ser
145                 150                 155                 160

Asp Thr Ala Glu Pro Thr Lys Ala Gly Arg Gly Ala Ser Gln Pro Pro
                165                 170                 175

Thr Pro Thr Pro Ala Ser Asp Ala Phe Gln Arg Lys Leu Glu Gly Cys
            180                 185                 190

Arg Phe Leu His Gly Tyr His Arg Phe Met His Ser Val Gly Arg Val
        195                 200                 205

Phe Ser Lys Trp Gly Glu Ser Pro Asn Arg Ser Arg Arg His Ser Pro
    210                 215                 220

His Gln Ala Leu Arg Lys Gly Val Arg Arg Thr Arg Pro Ser Arg Lys
225                 230                 235                 240

Gly Lys Arg Leu Met Thr Arg Gly Gln Leu Pro Arg
                245                 250

<210>  4
<211>  1880
<212>  DNA
<213>  homo sapiens

<400>  4
agccgagagg tgtcaccccc agcgggcgcg ggccggagca cgggcaccca gcatgggggt      60

actgctcaca cagaggacgc tgctcagtct ggtccttgca ctcctgtttc caagcatggc     120

gagcatggcg gctataggca gctgctcgaa agagtaccgc gtgctccttg gccagctcca     180

gaagcagaca gatctcatgc aggacaccag cagactcctg gacccctata tacgtatcca     240

aggcctggat gttcctaaac tgagagagca ctgcagggag cgccccgggg ccttccccag     300

tgaggagacc ctgagggggc tgggcaggcg gggcttcctg cagaccctca atgccacact     360

gggctgcgtc ctgcacagac tggccgactt agagcagcgc ctccccaagg cccaggattt     420

ggagaggtct gggctgaaca tcgaggactt ggagaagctg cagatggcga ggccgaacat     480

cctcgggctc aggaacaaca tctactgcat ggcccagctg ctggacaact cagacacggc     540

tgagcccacg aaggctggcc ggggggcctc tcagccgccc acccccaccc ctgcctcgga     600

tgcttttcag cgcaagctgg agggctgcag gttcctgcat ggctaccatc gcttcatgca     660

ctcagtgggg cgggtcttca gcaagtgggg ggagagcccg aaccggagcc ggagacacag     720

cccccaccag gccctgagga agggggtgcg caggaccaga ccctccagga aaggcaagag     780

actcatgacc aggggacagc tgccccggta gcctcgagag caccccttgc cggtgaagga     840

tgcggcaggt gctctgtgga tgagaggaac catcgcagga tgacagctcc cgggtcccca     900

aacctgttcc cctctgctac tagccactga gaagtgcact ttaagaggtg ggagctgggc     960

agacccctct acctcctcca ggctgggaga cagagtcagg ctgttgcgct cccacctcag    1020

ccccaagttc cccaggccca gtggggtggc cgggcgggcc acgcgggacc gactttccat    1080

tgattcaggg gtctgatgac acaggctgac tcatggccgg gctgactgcc cccctgcctt    1140

gctccccgag gcctgccggt ccttccctct catgacttgc agggccgttg cccccagact    1200

tcctcctttc cgtgtttctg aaggggaggt cacagcctga gctggcctcc tatgcctcat    1260

catgtcccaa accagacacc tggatgtctg ggtgacctca ctttaggcag ctgtaacagc    1320

ggcagggtgt cccaggagcc ctgatccggg ggtccaggga atggagctca ggtcccaggc    1380

cagccccgaa gtcgccacgt ggcctggggc aggtcacttt acctctgtgg acctgttttc    1440

tctttgtgaa gctagggagt tagaggctgt acaaggcccc cactgcctgt cggttgcttg    1500

gattccctga cgtaaggtgg atattaaaaa tctgtaaatc aggacaggtg gtgcaaatgg    1560

cgctgggagg tgtacacgga ggtctctgta aaagcagacc cacctcccag cgccgggaag    1620

cccgtcttgg gtcctcgctg ctggctgctc cccctggtgg tggatcctgg aattttctca 1680
cgcaggagcc attgctctcc tagagggggt ctcagaaact gcgaggccag ttccttggag 1740
ggacatgact aatttatcga tttttatcaa tttttatcag ttttatattt ataagcctta 1800
tttatgatgt atatttaatg ttaatattgt gcaaacttat atttaaaact tgcctggttt 1860
ctaaaaaaaa aaaaaaaaaa 1880

## Description

The present invention relates to an inhibitor of the oncostatin M receptor β or an inhibitor of an activator of the oncostatin M receptor β for use in the treatment and/or prevention of heart failure. The present invention also relates to a method of treating and/or preventing heart failure comprising administering a pharmaceutically effective amount of an inhibitor of the oncostatin M receptor β or an inhibitor of an activator of the oncostatin M receptor β to a subject in need thereof. Further, the present invention relates to methods of identifying a compound suitable as a lead compound and/or as a medicament for the treatment and/or prevention of heart failure.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Heart failure is one of the most frequent causes of death in western countries and is the common final manifestation of different diseases which becomes morphologically apparent in the reactivation of the fetal gene program (FGP), in myocardial remodelling and in the development of hypertrophy. In addition, during the transition to heart failure myocyte degeneration, partially compensated by hypertrophy, has been well documented in patients with dilative cardiomyopathy (DCM) as well as in pressure-overloaded human hearts (Hein *et al.* 2003; Heling *et al.* 2000; Schaper *et al.* 1995; Schaper *et al.* 1991; Sharma *et* al. 2004). While mechanical load/stress is considered to be a major mediator of these processes, there is increasing evidence that secreted morphogens, such as cytokines and growth factors, potently induce *in vitro* markers of mechanical load/stress such as the fetal gene program, hypertrophic responses as well as reorganization of the contractile and the cytoskeletal apparatus (Ebelt *et al.* 2007; Eppenberger-Eberhardt *et al*. 1997; Kubin *et al*. 1999) in the absence of mechanical load.

Over the past decades, there has been a shift in the conceptual paradigms that are used to explain the pathogenesis of heart failure (Seta *et al*. 1996). While the development of heart failure has been seen in the light of a hemodynamic disorder, there is increasing evidence that the syndrome of heart failure cannot be defined in terms of hemodynamic load. Concerns about the validity of the haemodynamic hypothesis led to the development of alternative models of heart failure namely the neurohormonal hypothesis (Packer 1995) and the cytokine hypothesis (Seta *et al*. 1996). According to these models, heart failure develops and progresses because neurohumoral factors or cytokines are activated by an initial injury and exert deleterious effects. The recognition of a potential correlation between progression to heart failure and increasing cytokine levels has led to an enormous research effort since this field is seen in the light of promising clinical benefits.

In addition, a growing body of evidence links the accumulation and activation of macrophages to the pathogenesis of heart failure (Hein *et al*. 2003; Heling *et al.* 2000; Schaper *et al.* 1991; Sharma *et al.* 2004). On the other hand, the production and secretion of cytokines by macrophages is considered to contribute to healing processes after an initial myocardial injury. Of special interest is oncostatin M (OSM), a powerful cytokine mainly restricted in its expression to macrophages in the adult tissue. OSM has been described as a potent morphogen with cardioprotective activities on myocytes (Ebelt *et al.* 2007). Due to the strong presence of oncostatin M-positive macrophages in a dog model of ischemia/reperfusion, a role of oncostatin M has been suggested in the healing process of myocardial infarction (Gwechenberger *et al.* 2004). Furthermore, myoblasts implanted into infarcted hearts release substantial amounts of OSM and are currently believed to exert beneficial effects (Ebelt *et al.* 2007). Thus, injury might lead to an invasion of monocytes into the cardiac tissue, where these cells contribute in the initial phase to tissue regeneration by secretion of OSM.

In animal models, receptor studies of the IL-6 class of cytokines, of which OSM is a member, support the idea of cardioprotective activities (Betz *et al.* 1998; Fischer and Hilfiker-Kleiner 2007; Hirota 1999). OSM binds and signals through a complex of OSM receptor ß and the coreceptor gp130; thus OSM shares gp130 as a signal transducing transmembrane protein with other cytokines of the IL-6 cytokine family. It has been demonstrated that postnatal inactivation of gp130 leads to cardiac defects (Betz *et al.* 1998). Furthermore, it has been shown in a mouse model of biomechanical load utilizing trans-aortic constriction that loss of gp130 leads to heart failure (Hirota 1999).

The current management of heart failure includes general measures, pharmacological therapy, mechanical devices and surgery. General measures in patients with heart failure include moderate sodium and fluids restriction, control of alcohol intake, weight reduction in overweight and obese patients, smoking cessation and regular exercise training. The pharmacological agents recommended in patients with heart failure include the angiotensin-converting enzyme inhibitors, beta-adrenoceptor antagonists, diuretics, aldosterone receptors antagonists and cardiac glycosides. Ventricular resynchronization therapy using bi-ventricular pacing is considered in patients with reduced ejection fraction and ventricular dyssynchrony and heart transplantation is considered in patients with end stage heart failure.

The technical problem underlying the present invention is the provision of alternative and/or improved means and methods for the development of medicaments for the treatment and/or prevention of heart failure.

The solution to this technical problem is achieved by providing the embodiments characterised in the claims.

Accordingly, the present invention relates to an inhibitor of the oncostatin M receptor β or an inhibitor of an activator of the oncostatin M receptor β for use in the treatment and/or prevention of heart failure.

The term "oncostatin M receptor β", also referred to herein as OSM receptor β, OSMR, OSMR-β or Oβ, relates to a receptor for oncostatin M, a pleitropic cytokine that belongs to the interleukin 6 group of cytokines. Signaling of oncostatin M occurs through cell surface receptors that contain the co-receptor gp130. The oncostatin M receptor β forms part of the type II receptor, which is composed of gp130 and OSMR-β, whereas the type I receptor is composed of gp130 and LIFR-β (leukemia inhibitory factor receptor β). The amino acid sequence derived from the human OSMR β cDNA sequence has been published in 1996 (Mosley *et al.* 1996). The complete human protein (SEQ ID NO: 1) and mRNA (SEQ ID NO:2) sequence are available under the accession number NM_003999 at the National Center for Biotechnology Information (NCBI).

The term "inhibitor" in accordance with the present invention refers to an inhibitor that reduces or abolishes the biological function or activity of a particular target protein. An inhibitor may perform any one or more of the following effects in order to reduce or abolish the biological function or activity of the protein to be inhibited: (i) the transcription of the gene encoding the protein to be inhibited is lowered, i.e. the level of mRNA is lowered, (ii) the translation of the mRNA encoding the protein to be inhibited is lowered, (iii) the protein performs its biochemical function with lowered efficiency in the presence of the inhibitor, and (iv) the protein performs its cellular function with lowered efficiency in the presence of the inhibitor.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Compounds of class (iii) interfere with molecular function of the protein to be inhibited, such as receptor signalling activity and activation of downstream target molecules. Accordingly, active site binding compounds are envisaged. Class (iv) includes compounds which do not necessarily bind directly to target, but still interfere with its function or activity, for example by binding to and/or inhibiting the function or inhibiting expression of members of a pathway which comprises the target. These members may be either upstream or downstream of the target within said pathway. For example, such compounds may alter the affinity or rate of binding of a known ligand to the receptor or compete with a ligand for binding to the receptor or displace a ligand bound to the receptor.

The inhibitor, in accordance with the present invention, may in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Methods for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to either selectively or permanently elevate the level of the encoded inhibitor in any cell or a subset of selected cells of the recipient. Thus, a tissue- and/or time-dependent expression of the inhibitor can be achieved, for example restricted to cells of the myocardium. In a preferred embodiment, the inhibitor is therefore a myocardial-specific inhibitor.

The term "inhibitor of oncostatin M receptor ß" in accordance with the present invention refers to an inhibitor that reduces the biological function or activity of the oncostatin M receptor β. Biological function or activity denotes in particular any known biological function or activity of the oncostatin M receptor β including those elucidated in accordance with the present invention. Examples of said biological function or activity are the activation of the B-Raf/MEK/Erk signaling cascade, the induction of the fetal gene program, sarcomere degradation as well as fibrosis leading to an impaired heart function. All these functions or activities can be tested for either using any of a variety of standard methods known in the art, such as echocardiography, heart catheter and cardiac magnetic resonance tomography or on the basis of the teachings of the examples provided below, optionally in conjunction with molecular techniques such as RT-PCR or with the teachings of the documents cited therein.

In a preferred embodiment, the inhibitor reduces the biological function or activity of the oncostatin M receptor β by at least 50%, preferably by at least 75%, more preferred by at least 90% and even more preferred by at least 95% such as at least 98% or even by 100%. The term reduction by at least, for example 75%, refers to a decreased biological function or activity such that the oncostatin M receptor β loses 75% of its function or activity and, consequently, has only 25% of the function or activity remaining as compared to the oncostatin M receptor β that is not inhibited.

The function of any of the inhibitors referred to in the present invention may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of binding of potential inhibitors can be effected in, for example, any binding assay, preferably biophysical binding assay, which may be used to identify binding of test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay.

In cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 4 µl of 5x AMV-RT buffer, 2 µl of oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique.

The term "inhibitor of an activator of the oncostatin M receptor β" as used in accordance with the present invention refers to any inhibitor that does not directly interact with the oncostatin M receptor β but with molecules that directly or indirectly activate one or more of the biological functions or activities of the oncostatin M receptor ß and preferably one or more of those functions or activities referred to above or elsewhere in this specification. The inhibition values referred to above for the inhibitor of the oncostatin M receptor ß apply *mutatis mutandis* to the inhibitor of an activator of the oncostatin M receptor ß.

For example, the inhibitor of an activator of the oncostatin M receptor ß may be any compound that reduces the amount of the ligand oncostatin M available for binding to the receptor. Such a compound may act on oncostatin M directly, such as for example by reducing its expression levels or its binding abilities to the receptor, or it may act on oncostatin M-producing cells, such as macrophages. Thus, also encompassed by the term inhibitor of an activator of the oncostatin M receptor ß are compounds that either reduce the number of macrophages or that interfere with oncostatin M production and/or release by macrophages.

The term "heart failure", as used in accordance with the present invention, relates to a clinical syndrome in which an abnormality of cardiac heart structure or function is responsible for the inability of the heart to fill with or eject blood at a rate commensurate with the requirements of the metabolising tissues (E. Braunwald, Heart failure and cor pulmonale, in *Harrison's Principles of Internal Medicine,* 16^{th} ed (2005) 1367). Heart failure includes abnormalities during systole (systolic heart failure) and/or diastole (diastolic heart failure), abnormalities in the left and/or right ventricle, chronic and/or acute heart insufficiency, low-output and/or high-output heart failure. Heart failure includes, but is not limited to, conditions and diseases such as hypertrophic cardiomyopathy; arrhythmogenic right ventricular dysplasia cardiomyopathy; left ventricular noncompaction cardiomyopathy; cardiomyopathies associated with conduction defect and/or ion channel disorders; primary and secondary dilated cardiomyopathy; primary restrictive nonhypertrophied cardiomyopathy, idiopathic cardiomyopathy; stress ("Tako-Tsubo") cardiomyopathy; tachycardia-induced cardiomyopathy; pregnancy-related cardiomyopathy; toxic cardiomyopathy such as caused by alcohol abuse, cocaine use, ephedrine use, chemotherapeutic agent, radiation; restrictive cardiomyopathy secondary to myocardial infiltrative or storage diseases, such as amyloidosis, Gaucher disease, Hurler's disease, Hunter's disease, hemochromatosis, Fabry's disease, glycogen storage disease, Niemann-Pick disease; cardiomyopathy related to endocrine and/or metabolic disorders such as thyroid disorders, hyperparathyroidism, pheochromocytoma, acromegaly, obesity cardiomyopathy; cardiomyopathy related to neuromuscular and/or neurologic disorders such as Friedreich's ataxia, muscular dystrophy, myotonic dystrophy, neurofibromatosis, tuberous sclerosis; cardiomyopathy secondary to nutritional deficiencies such as beriberi, pellagra, kwashiorkor, selenium deficiency, carnitine deficiency as well as cardiomyopathy secondary to autoimmune and/or collagen vascular diseases such as secondary to systemic lupus erythematosus, dermatomyositis, rheumatoid arthritis, scleroderma, polyarteritis nodosa. Furthermore, heart failure may result from a large number of diseases or conditions, including, but not limited to, ischemic heart disease; valvular heart disease; hypertensive cardiomyopathy; inflammatory and/or infectious myocarditis, such as viral myocarditis, bacterial myocarditis, rickettsial myocarditis, fungal myocarditis, parasitic myocarditiy (Chagas disease, toxoplasmosis), sarcoidosis; pulmonary hypertension or obstructive sleep apnea (Maron, et al (2006), Circulation 113:1807; E. Braunwald, E. Heart failure and cor pulmonale, in Harrison's Principles of Internal Medicine, 16th ed (2005) 1367).

Preferably, the inhibitor of the present invention is comprised in a pharmaceutical composition, optionally further comprising a pharmaceutically acceptable carrier, excipient and/or diluent.

The term "pharmaceutical composition", as used herein, relates to a composition for administration to a patient, preferably a human patient. The pharmaceutical composition of the invention comprises at least one, such as at least two, e.g. at least three, in further embodiments at least four such as at last five of the above mentioned inhibitors. The invention also envisages mixtures of inhibitors of the oncostatin M receptor ß or of inhibitors of an activator of the oncostatin M receptor ß. In cases where more than one inhibitor is comprised in the pharmaceutical composition it is understood that none of these inhibitors has any essentially inhibitory effect on the other inhibitors also comprised in the composition.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the bloodstream such as a coronary artery or directly into the respective tissue. The compositions of the invention may also be administered directly to the target site, e.g., by biolistic delivery to an external or internal target site, like the heart. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize heart failure. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

In accordance with the present invention, it was surprisingly found that oncostatin M acts as a crucial regulator of pathological remodelling, such as the activation of the fetal gene program (FGP), fibrosis and loss of sarcomeric structure in the heart. This pathological remodelling leads to changes in the phenotype of cardiomyocytes, i.e. a dedifferentiation of cardiomyocytes and the loss of contractile function results finally in heart failure.

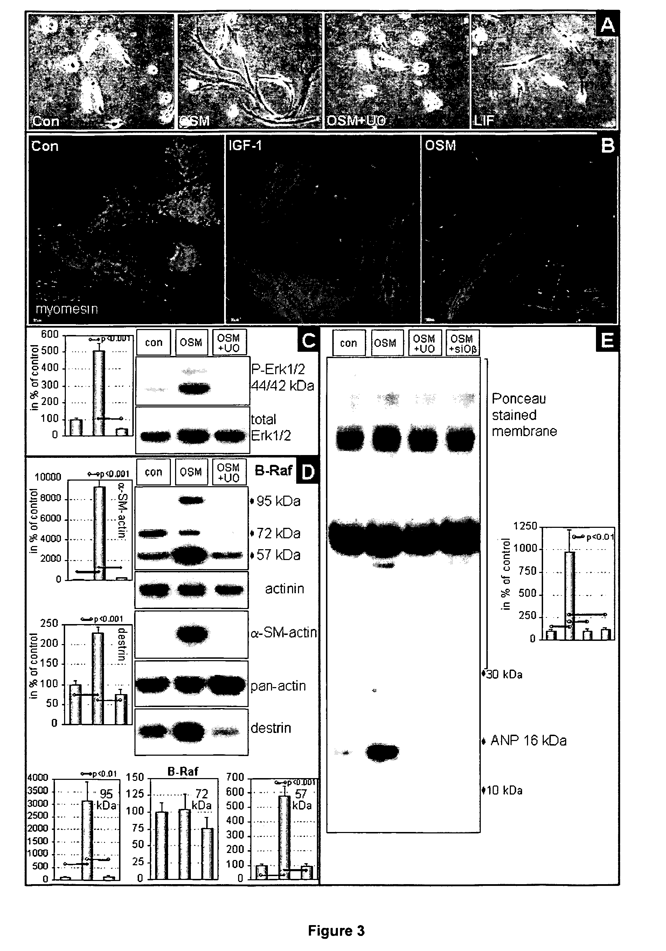

Thus, whereas it was previously believed that OSM has mainly cardio-protective activities (Ebelt *et al.* 2007; Gwechenberger *et al.* 2004), it was now surprisingly found in accordance with the present invention that the heart's myocardial function is impaired upon prolonged exposure to OSM. This effect of OSM on cardiomyocytes can also be observed in culture, since a prolonged exposure leads to sarcomeric loss reminiscent to the transition to heart failure, as shown in figure 3B of the present invention.

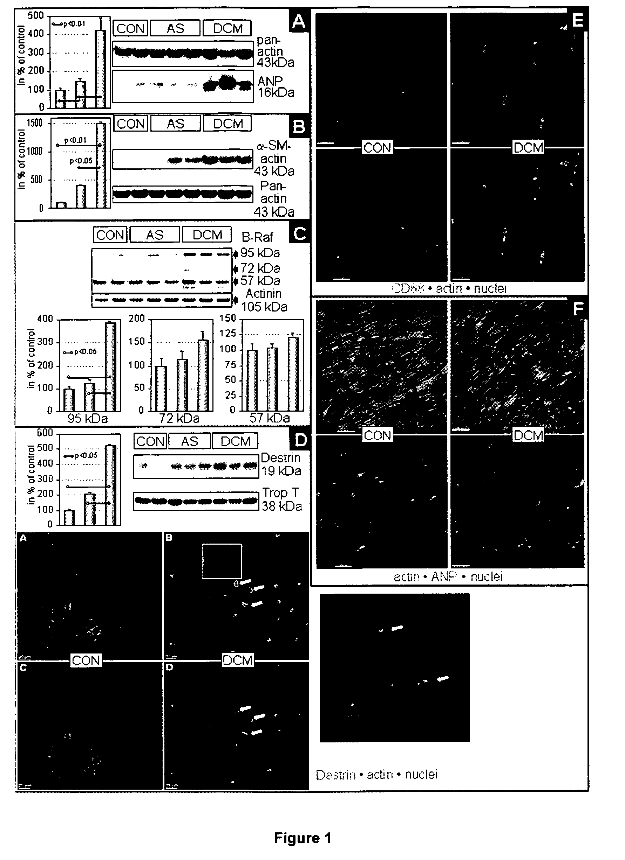

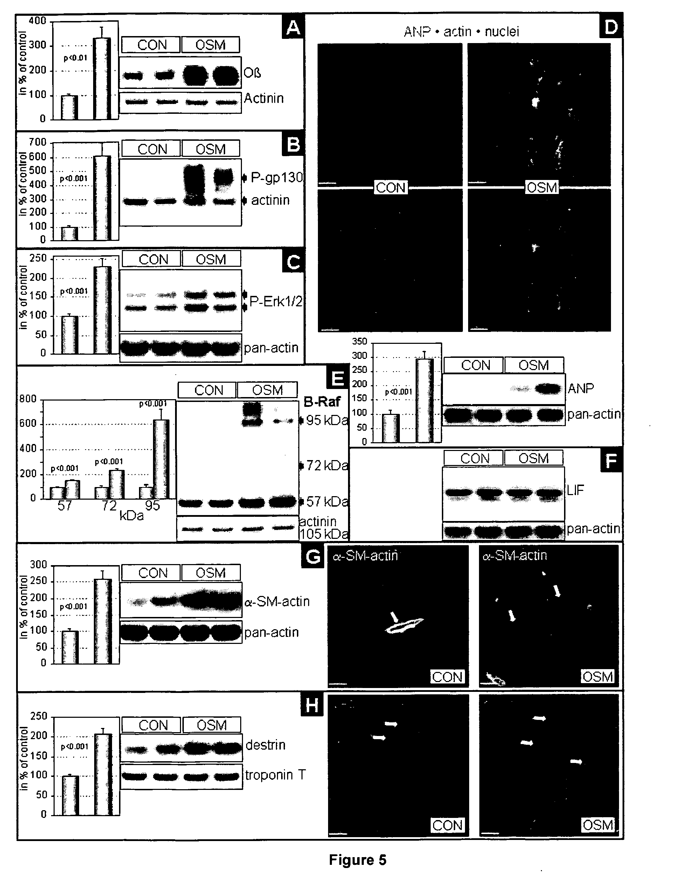

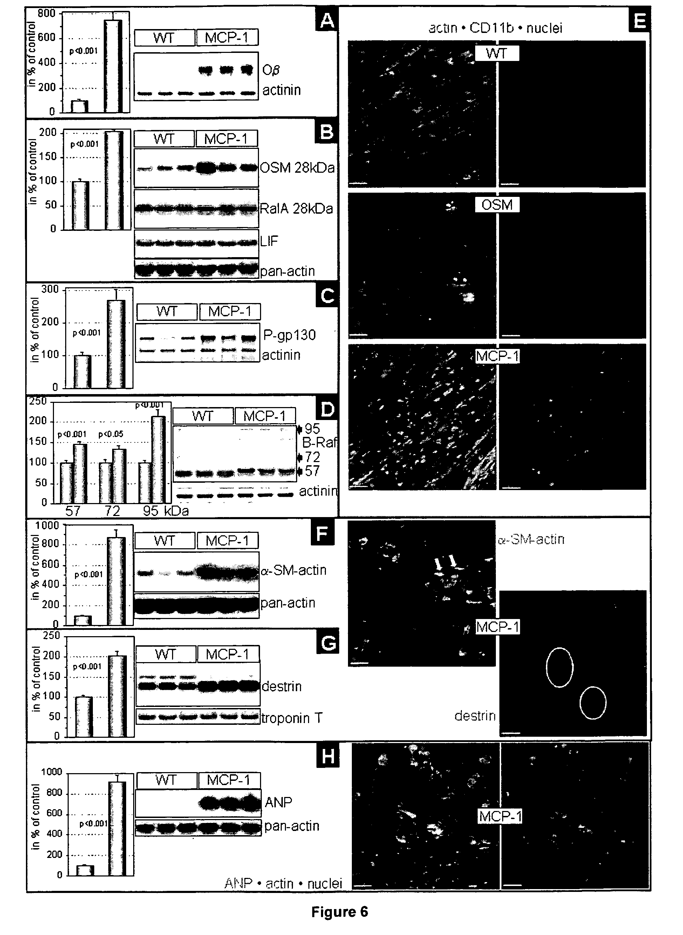

It was further found in accordance with the present invention that the activation of fetal gene program (FGP) and pathological remodelling in dilative cardiomyopathy (DCM) upon prolonged periods of exposure to OSM are mediated via the OSM receptor β. OSM was found to exert this action in mice and isolated cardiomyocytes via stimulation of the MEK/Erk pathway. Furthermore, OSM and its receptor were found to be increased in patients with FGP (Fig.1) and it was additionally shown in a mouse model of myocarditis that the activation of the OSM receptor complex leads to DCM (Fig.6). Furthermore, injection of OSM into mice also resulted into the heart failure phenotype of DCM (Fig.5). Inhibition of the MEK/ERK pathway by chemical inhibitors and targeting the OSM receptor by siRNA or specific antibodies was shown to be sufficient to prevent FGP.

The first visible morphological change of OSM treated cardiomyocytes is cellular outgrowth from the area of the intercalated disc which finally results in large extensions. As a result, cultured cardiomyocytes show enhanced cell-cell contacts and increased viability. During cell growth a reorganisation of myocyte structure involves loss of sarcomeric structure, re-expression of α-SM actin, depolymerisation mediated by actin dynamising proteins such as destrin and polymerisation of actin visible in α-SM actin positive cardiomyocytes. Concomitantly, accumulation of ANP, a classical marker of cardiomyocyte dedifferentiation, and the re-expression of the 95 kDa B-Raf isoform are observed. The function of dedifferentiation in DCM patients is unknown. However, dedifferentiation and loss of contractile material has been reported in hibernating myocardium (Ausma *et al.* 1995; Ausma *et al.* 1998) and in infarct border zones (Dispersyn *et al.* 2002) leading to the conclusion that dedifferentiated cardiomyocytes are more ischemia tolerant (Ausma *et al.* 1995; Ausma *et al.* 1998; Dispersyn *et al.* 2002). Furthermore, it has been demonstrated that mice with cardiac restricted over-expression of MCP-1, the transgenic model used in the examples, showed an increased resistance to periods of ischemic/ reperfusion (Martire *et al.* 2003) indicating that dedifferentiated cardiomyocytes are more resistant to hypoxia. Whereas this dedifferentiation might have beneficial effects in the short term, it has been shown here that it is detrimental in the long term, as these transgenic mice rarely survived 6 months and died because of heart failure (Kolattukudy *et al.* 1998). In accordance with the present invention, it was shown by magnetic resonance imaging and Western Blot analysis that the morphological changes in the contractile structure coincide with the fetal gene program and impaired cardiac performance. It was further demonstrated that the block of the oncostatin M receptor β did not impair the left ventricular enlargement, but abolished the reduction of left ventricular contraction. Furthermore, Western Blot analysis revealed that the block of Oβ led also to a reduction in FGP. Thus, the oncostatin M receptor β was shown herein to play a key role in the transition to heart failure.

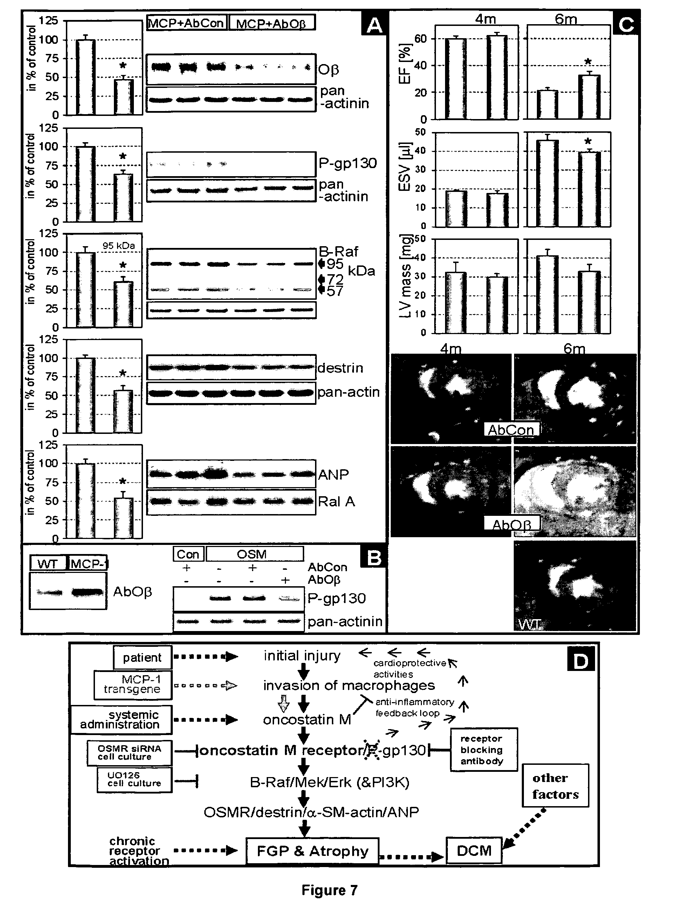

Figure 7D provides a summary of the above discussed effects. Without wishing to be bound by any theory, it is hypothesised that, initially, invading macrophages release OSM and contribute thereby to repair mechanisms in the damaged myocardium. OSM exerts in this phase anti-inflammatory activities through blocking further macrophage recruitment and halts chronical invasion. However, in the chronic phase macrophages contribute to the progression of heart failure through the secretion of OSM, which was shown herein to be important in the pathogenesis of heart failure such as cellular signalling, FGP, degenerative responses as well as reorganisation of the contractile and cytoskeletal apparatus. Its powerful activities are mediated through OSMRβ, which is not utilised by the other members of the IL-6 cytokine family. Accordingly, therapeutic agents preventing the activation of the OSM receptor β provide a promising approach for therapies in patients with heart failure. In addition, a targeted inactivation of the OSM receptor β provides a specific approach for overcoming the detrimental effects of OSM in the development of heart failure without interfering with the beneficial, i.e. cardio-protective effects of cytokines acting through the gp130 co-receptor.

The present invention further relates to a method of treating and/or preventing heart failure comprising administering a pharmaceutically effective amount of an inhibitor of the oncostatin M receptor β or an inhibitor of an activator of the oncostatin M receptor ß to a subject in need thereof.

Also with regard to this embodiment, it is preferred that the inhibitor is comprised in a pharmaceutical composition as defined above.

In a preferred embodiment of the inhibitor or the method of the invention, the inhibitor is an antibody or a fragment or derivative thereof, an aptamer, an siRNA, an shRNA, a miRNA, a ribozyme, an antisense nucleic acid molecule, modified versions of these inhibitors or a small molecule.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of the oncostatin M receptor β or an epitope of an activator of the oncostatin M receptor β (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "polypeptide" as used herein describes a group of molecules consisting of more than 30 amino acids. The group of peptides and polypeptides are referred to together with the term "(poly)peptide". Also encompassed by the term "(poly)peptide" are proteins as well as fragments of proteins. The term "fragment of protein" in accordance with the present invention refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.

It is also well known that (poly)peptides are not always entirely linear. For instance, (poly)peptides may be branched as a result of ubiquitination, and they may be circular, with or without branching, generally as a result of post-translation events, including natural processing event and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of the oncostatin M receptor β or an inhibitor of an activator of the oncostatin M receptor β.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

The term "modified versions of these inhibitors" in accordance with the present invention refers to versions of the inhibitors that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiffs bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

A "small molecule" as used herein may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays.

In another preferred embodiment of the inhibitor or the method of the invention, the activator of the oncostatin M receptor β is oncostatin M. The term "oncostatin M", as used herein, refers to a multifunctional cytokine that was first identified in 1986 as a polypeptide that inhibited proliferation of several cancer cell lines (Zarling *et al.* 1986). The cDNA sequence of human OSM and its derived amino acid sequence has been published in 1989 (Malik, N *et al*. 1989). The complete human protein (SEQ ID NO: 3) and mRNA (SEQ ID NO:4) sequence are available under the accession number NM_020530 at the National Center for Biotechnology Information (NCBI). It is structurally related to a family of hematopoietic and neurotrophic cytokines whose founding member was interleukin 6 (IL-6). Further members include leukemia inhibitory factor (LIF), cardiotrophin (CT) and interleukin 11(Mire-Sluis & Thorpe 1998). In contrast to the other members, very few cell types, such as macrophages and activated T cells, have been shown to synthesise and secrete OSM (Mire-Sluis & Thorpe 1998). Despite its restricted production, OSM has been shown to act on many cells and to elicit a variety of diverse responses in broad ranging processes such as angiogenesis (Vasse *et al.* 1999), cholesterol metabolism, atherosclerosis, inflammation, cancer, AIDS, hematopoiesis and neurotrophic effects (Mire-Sluis & Thorpe 1998). Human OSM is a single-copy gene located on chromosome 22q12 (Mire-Sluis & Thorpe 1998). Human OSM can signal via two different receptors, the OSM receptor β (OSMRβ) and the LIF receptor (LIFR). Both receptor subtypes are present in the heart and complex with gp130 receptor, which is an essential signalling component of the IL-6 family of cytokines. Whereas blockade of oncostatin M could potentially lead to reduced LIFR signalling, the main activation of LIFR occurs through the more abundant leukemia inhibitory factor and cardiotrophin. Thus, the inhibition of oncostatin M is expected to block OSMRβ signalling, while only affecting minor changes in the LIFR activation. In mice, on the other hand, OSM is incapable of utilising the mouse LIFR complex but binds and signals through a complex of OSMRβ and gp130 (Ichihara *et al.* 1997).

The present invention further relates to a method of identifying an inhibitor of the oncostatin M receptor β or an inhibitor of an activator of the oncostatin M receptor β suitable as a lead compound and/or as a medicament for the treatment and/or prevention of heart failure, comprising the steps of: (a) determining the level of oncostatin M receptor β protein or oncostatin M receptor β transcript in a cell; (b) contacting said cell or a cell of the same cell population with a test compound; (c) determining the level of oncostatin M receptor β protein or oncostatin M receptor β transcript in said cell after contacting with the test compound; and (d) comparing the level of oncostatin M receptor β protein or oncostatin M receptor β transcript determined in step (c) with the oncostatin M receptor β protein or oncostatin M receptor β transcript level determined in step (a), wherein a decrease of oncostatin M receptor β protein or oncostatin M receptor β transcript level in step (c) as compared to step (a) indicates that the test compound is an inhibitor of the oncostatin M receptor β or an inhibitor of an activator of the oncostatin M receptor β suitable as a lead compound and/or as a medicament for the treatment and/or prevention of heart failure.

This embodiment relates to a cellular screen, wherein inhibitors may be identified which exert their inhibitory activity by interfering with the expression of oncostatin M receptor β, either by affecting the stability of oncostatin M receptor β protein or transcript (mRNA) or by interfering with the transcription or translation of oncostatin M receptor β.

The inhibitor can be any of the inhibitors defined above, i.e. an antibody or a fragment or derivative thereof, an aptamer, a siRNA, a shRNA, a miRNA, a ribozyme, an antisense nucleic acid molecule, modified versions of these inhibitors or a small molecule. The inhibitor may further be, for example a (poly)peptide such as a soluble peptide, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids or phosphopeptides (e.g., members of random and partially degenerate, directed phosphopeptide libraries, see, e.g., Songyang et al. (1993) Cell 72: 767-778).

The term "said cell or a cell of the same cell population" as used herein refers either to the cell used in step (a) or to a cell being of the same origin as the cell of step (a) and that is identical in its characteristics to the cell of (a). Furthermore, this term also encompasses cell populations, such as for example homogenous cell populations consisting of cells having identical characteristics, and, thus, is not restricted to single cell analyses.

As described hereinabove, the oncostatin M receptor ß plays a key role in the transition to heart failure. Therefore, the use of oncostatin M receptor ß as a target for the discovery of inhibitors suitable for the treatment and/or prevention of heart failure is also encompassed by the present invention. It is envisaged that a decrease of expression levels of oncostatin M receptor ß conferred by an inhibitor as described above may contribute to protection from heart failure and may ameliorate conditions associated therewith, as described above. Accordingly, measurement of the oncostatin M receptor ß protein or oncostatin M receptor β transcript level may be used as a readout of the above-described assay.

For example, the above-mentioned cell may exhibit a detectable level of oncostatin M receptor β protein or oncostatin M receptor ß transcript before contacting with the test compound and the level of oncostatin M receptor ß protein or oncostatin M receptor ß transcript may be lower or undetectable after contacting the cell with the test compound, indicating an inhibitor suitable for the treatment and/or prevention of heart failure or as a lead compound for the development of a compound for the treatment of heart failure. Preferably, the level of oncostatin M receptor ß protein or oncostatin M receptor ß transcript after contacting the cell with the test compound is reduced by, for example, at least 10, at least 20, at least 30, at least 40 or at least 50% as compared to the level of oncostatin M receptor ß protein or oncostatin M receptor β transcript before contacting the cell with the test compound. More preferably, the level of oncostatin M receptor β protein or oncostatin M receptor ß transcript after contacting the cell with the test compound is reduced by, for example, at least 60, at least 70, at least 80, at least 90 or at least 95% as compared to the level of oncostatin M receptor β protein or oncostatin M receptor β transcript before contacting the cell with the test compound. Most preferably, the level of oncostatin M receptor β protein or oncostatin M receptor β transcript after contacting the cell with the test compound is reduced by 100% as compared to the level of oncostatin M receptor β protein or oncostatin M receptor β transcript before contacting the cell with the test compound. The term "the level of oncostatin M receptor β protein or oncostatin M receptor β transcript is reduced by (at least)... %" refers to a relative decrease compared to the level of oncostatin M receptor β protein or oncostatin M receptor β transcript before contacting the cell with the test compound. For example, a reduction of at least 40% means that after contacting the cell with the test compound the remaining level of oncostatin M receptor β protein or oncostatin M receptor β transcript is only 60% or less as compared to the level of oncostatin M receptor β protein or oncostatin M receptor β transcript before contacting the cell with the test compound. A reduction by 100% means that no detectable level of oncostatin M receptor β protein or oncostatin M receptor β transcript remains after contacting the cell with the test compound.

Measurements of protein levels as well as of transcript level can be accomplished in several ways, as described above.

In a preferred embodiment, the method is carried out *in vitro. In vitro* methods offer the possibility of establishing high-throughput assays, as described above.

The present invention also relates to a method of identifying an inhibitor of the oncostatin M receptor β or an inhibitor of an activator of the oncostatin M receptor β suitable as a lead compound and/or as a medicament for the treatment and/or prevention of heart failure, comprising the steps of: (a) determining the level of activity of an oncostatin M receptor β target molecule in a cell containing oncostatin M receptor β protein; (b) contacting said cell or a cell of the same cell population with a test compound; (c) determining the level of activity of the oncostatin M receptor β receptor target molecule in said cell after contacting with the test compound; and (d) comparing the level of activity of the oncostatin M receptor β target molecule determined in step (c) with the level of activity of the oncostatin M receptor β target molecule determined in step (a), wherein a decreased activity of the target molecule in step (c) as compared to step (a) indicates that the test compound is an inhibitor of the oncostatin M receptor β or an inhibitor of an activator of the oncostatin M receptor β suitable as a lead compound and/or as a medicament for the treatment and/or prevention of heart failure.

This embodiment relates to a cellular screen, wherein inhibitors may be identified which exert their inhibitory activity by physically interacting with the oncostatin M receptor β, or alternatively (or additionally) by functionally interacting with the oncostatin M receptor β, i.e., by interfering with the pathway(s) present in the cells employed in the cellular assay. Furthermore, such compounds may, as described above, alter the affinity or rate of binding of a known ligand, such as OSM, to the receptor or compete with a ligand for binding to the receptor or displace a ligand bound to the receptor. As a result, the biological activity of the oncostatin M receptor β is altered either directly or indirectly, which can be measured as an altered level of activity of an oncostatin M receptor β target molecule.

The term "oncostatin M receptor β target molecule" refers to molecules that are affected by oncostatin M receptor β activity. For example, the oncostatin M receptor β target molecule can be a molecule affected by oncostatin M receptor β activity as a result of the downstream signalling of this receptor. Downstream signalling refers to the modulation (e.g., stimulation or inhibition) of a cellular function/activity upon binding of a ligand to the receptor. Examples of such functions include mobilization of intracellular molecules that participate in a signal transduction pathway, such as for example the B-Raf/MEK/Erk and Erk5 signalling pathway or that result in a change in the amount of intracellular molecules, for example ANP, α-smooth muscle actin, sarcomeric actin, destrin, sarcomeric and non-sarcomeric actinin, alteration in the structure of a cellular component, such as for example the sarcomere, cell differentiation and cell survival.

The "level of activity" of an oncostatin M receptor β target molecule may be altered due to a change in the biological activity of the oncostatin M receptor β target molecule. Alternatively, the "level of activity" may also be altered by reducing or increasing the expression level of the oncostatin M receptor β target molecule.

Assays determining the expression of genes that are up- or down-regulated in response to the receptor protein dependent signal cascade can be employed. For example, any of the methods described above for determining the expression of a protein on the protein or the nucleic acid level may be used. Furthermore, the regulatory region of target genes may be operably linked to a marker that is easily detectable, such as for example luciferase, green fluorescent protein (GFP), yellow fluorescent protein (YFP), red fluorescent protein (RFP), cyan fluorescent protein (CFP) or β-galactosidase. Alternatively, for target molecules that have an altered activity upon phosphorylation, the phosphorylation level of said molecules may be measured.

In a preferred embodiment, the oncostatin M receptor β target molecule is selected from JAK1, STAT1, STAT3, STAT5, MEK1/2, Erk1/2, Erk5, SOCS3 and p38.

All of these molecules are defined in accordance with the present invention in the same manner as known in the prior art and the common general knowledge of the skilled person.

"JAK1", in accordance with the present invention, relates to Janus kinase 1 (UniProtKB Database ID: P23458; Wilks *et al.* 1991), which belongs to a class of protein-tyrosine kinases (PTK) characterised by the presence of a second phosphotransferase-related domain immediately N-terminal to the PTK domain. The second phosphotransferase domain bears all the hallmarks of a protein kinase, although its structure differs significantly from that of the PTK and threonine/serine kinase family members. JAK1 is a large, widely expressed membrane-associated phosphoprotein. JAK1 is involved in the interferon-alpha/beta and -gamma signal transduction pathways and couples cytokine ligand binding to tyrosine phosphorylation of various known signaling proteins and of a unique family of transcription factors termed the signal transducers and activators of transcription, or STATs.

In accordance with the present invention, "STAT" refers to the family of signal transducer and activator of transcription. In response to cytokines and growth factors, STAT family members are phosphorylated by the receptor associated kinases, and then form homo- or heterodimers that translocate to the cell nucleus where they act as transcription activators. "STAT1" refers to the signal transducer and activator of transcription 1 (UniProtKB Database ID: P42224; Schindler *et al*. 1992). This protein can be activated by various ligands including interferon-alpha, interferon-gamma, EGF, PDGF and IL6. This protein mediates the expression of a variety of genes, which is thought to be important for cell viability in response to different cell stimuli and pathogens. Two alternatively spliced transcript variants encoding distinct isoforms have been described.

"STAT3" refers to the signal transducer and activator of transcription 3 (UniProtKB Database ID: P40763; Akira *et al.* 1994). This protein is activated through phosphorylation in response to various cytokines and growth factors including interferons (IFNs), epidermal growth factor (EGF), interleukin-5 (IL5), interleukin-6 (IL6), hepatocyte growth factor (HGF), leukemia inhibitory factor (LIF) and bone morphogenetic protein 2 (BMP2). STAT3 mediates the expression of a variety of genes in response to cell stimuli, and thus plays a key role in many cellular processes such as cell growth and apoptosis. The small GTPase Rac1 has been shown to bind and regulate the activity of this protein. Protein inhibitor of activated STAT 3 (PIAS3) is a specific inhibitor of this protein. Three alternatively spliced transcript variants encoding distinct isoforms have been described.

"STAT5" refers to the signal transducer and activator of transcription 5. STAT5A (UniProtKB Database ID: P42229; Hou *et al.* 1995) is activated by, and mediates the responses of many cell ligands, such as interleukin-2 (IL2), interleukin-3 (IL3), interleukin-7 (IL7), Granulocyte-macrophage colony-stimulating factor (GM-CSF), erythropoietin, thrombopoietin, and different growth hormones. Activation of this protein in myeloma and lymphoma associated with a TEUJAK2 gene fusion is independent of cell stimulus and has been shown to be essential for the tumorigenesis. The mouse counterpart of this gene is found to induce the expression of BCL2L1/BCL-X(L), which suggests the anti-apoptotic function of this gene in cells. STAT5B (UniProtKB Database ID: P51692; Lin *et al.* 1996) mediates the signal transduction triggered by various cell ligands, such as interleukin-2 (IL2), interleukin-4 (IL4), macrophage colony-stimulating factor (CSF1), and different growth hormones. It has been shown to be involved in diverse biological processes, such as TCR signaling, apoptosis, adult mammary gland development, and sexual dimorphism of liver gene expression. This gene was found to fuse to retinoic acid receptor-alpha (RARA) gene in a small subset of acute promyelocytic leukemias (APLL). The dysregulation of the signaling pathways mediated by this protein may be the cause of the APLL.

"MEK" in accordance with the present invention refers to mitogen-activated protein kinase kinase, which is a kinase enzyme that phosphorylates mitogen-activated protein kinase. It is also known as MAP2K and is classified as EC 2.7.12.2. "MEK1" according to the present invention, relates to mitogen activated protein kinase kinase 1 (UniProtKB Database ID: Q02750; Seger *et al*. 1992) whereas "MEK2" relates to mitogen activated protein kinase kinase 2 (UniProtKB Database ID: P36507; Zheng *et al.* 1993). MEK1/2 activate the ERK1/2 MAPK enzymes by phosphorylating both residues within the threonine - glutamic acid - tyrosine (TEY) motif in the activation loop.

"Erk", as used herein, relates to the extracellular signal-regulated kinases or classical mitogen-activated protein kinase (MAPK). ERKs are widely expressed protein kinases and intracellular signaling molecules, which are involved in functions including the regulation of meiosis, mitosis, and postmitotic functions in differentiated cells. Many different stimuli, including growth factors, cytokines, virus infection, ligands for heterotrimeric G protein-coupled receptors, transforming agents, and carcinogens, activate the ERK pathway. "ERK1" according to the present invention, relates to the extracellular signal-regulated kinases 1 (UniProtKB Database ID: P27361; Charest *et al*. 1993) whereas "ERK2" relates to the extracellular signal-regulated kinases 2 (UniProtKB Database ID: P28482; Owaki *et al.* 1992). In the MAPK/ERK pathway, Ras activates c-Raf, followed by mitogen-activated protein kinase kinase (MKK) and then MAPK1/2. Ras is typically activated by growth hormones through receptor tyrosine kinases and GR62/SOS, but may also receive other signals. ERKs are known to activate many transcription factors and some downstream protein kinases. Disruption of the ERK pathway is common in cancers, especially Ras, c-Raf and receptors such as the human epidermal growth factor receptor 2 (HER2).

"Erk5", as used herein, is a recently identified new member of the MAPK family originally described as big mitogen-activated kinase 1 or shortly as BMK1 (UniProtKB Database ID: Q13164; Lee *et al.* 1995). MEKK2 or MEKK3 and subsequent MEK5 are reported to cause Erk5 activation. Various forms of stress such as oxidative stress and hyperosmolarity are involved in the activation of Erk5. A recent report emphasizes the role of Erk5 in cardiovascular development and diseases (Wang 2007).

"SOCS3", in accordance with the present invention, relates to the suppressor of cytokine signaling 3 (UniProtKB Database ID: 014543; Minamoto *et al.* 1997). This protein is a member of the STAT-induced STAT inhibitor (SSI) family, also known as suppressor of cytokine signaling (SOCS) family. SSI family members are cytokine-inducible negative regulators of cytokine signaling. The expression of this gene is induced by various cytokines, including interleukin-6 (IL6), interleukin-10 (IL10), and interferon (IFN)-γ. SOCS3 can bind to janus kinase 2 (JAK2) and inhibit the activity of JAK2. Studies of the mouse counterpart of SOCS3 suggested a roles in the negative regulation of fetal liver haematopoiesis, and placental development.

"p38", as used herein, relates to a class of mitogen-activated protein kinases which are responsive to stress stimuli, such as cytokines, ultraviolet irradiation, heat shock, and osmotic shock, and are involved in cell differentiation and apoptosis. Four isoforms of p38 MAP kinase, p38-α (MAPK14; UniProtKB Database ID: Q16539; Lee *et al.* 1994), p38-β (MAPK11; UniProtKB Database ID: Q15759; Jiang *et al.* 1996), p38-γ (MAPK12 or ERK6; UniProtKB Database ID: P53778; Lechner *et al.* 1996) and p38-δ (MAPK13 or SAPK4; UniProtKB Database ID: 015264; Goedert *et al.* 1997) have been identified. Similar to the SAPK/JNK pathway, p38 MAP kinase is activated by a variety of cellular stresses including osmotic shock, inflammatory cytokines, lipopolysaccharides (LPS), ultraviolet light and growth factors. MKK3 and SEK activate p38 MAP kinase by phosphorylation at Thr180 and Tyr182. Activated p38 MAP kinase has been shown to phosphorylate and activate MAPKAP kinase 2 and to phosphorylate the transcription factors Activating transcription factor 2 (ATF-2), Mac and myocyte enhancer factor-2 (MEF2).

Intracellular levels and activity of these molecules can be determined using methods well known in the art, for example using any of the methods described above.

In another preferred embodiment of the methods of the invention, said cell comprising the oncostatin M receptor β is the cardiomyocyte.

The term "cardiomyocyte", as used herein, refers to cardiac myocytes containing sarcomeric structures.

In another preferred embodiment, the methods of the invention further comprise optimising the pharmacological properties of a compound identified as lead compound.

The identified so-called lead compounds may be optimized to arrive at a compound which may be used in a pharmaceutical composition. Methods for the optimization of the pharmacological properties of compounds identified in screens, the lead compounds, are known in the art.

In a more preferred embodiment, the optimisation comprises modifying the compound to achieve: (i) modified spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (I) conversion of alkyl substituents to cyclic analogues, or (m) derivatisation of hydroxyl groups to ketales, acetales, or (n) N-acetylation to amides, phenylcarbamates, or (o) synthesis of Mannich bases, imines, or (p) transformation of ketones or aldehydes to Schiff's bases, oximes, acetales, ketales, enolesters, oxazolidines, thiazolidines or combinations thereof.

The various steps recited above are generally known in the art, as described above.

In another preferred embodiment of the inhibitor or the method of the invention, the heart failure is selected from dilated cardiomyopathy, myocarditis, inflammatory cardiomyopathy and ischemic cardiomyopathy.

In accordance with the present invention, the term "dilated cardiomyopathy" refers to the most common form of cardiomyopathy and is a condition in which the heart becomes weakened and enlarged, and cannot pump blood efficiently. The decreased heart function can affect the lungs, liver, and other body systems. In dilated cardiomyopathy a portion of the myocardium is dilated, often without any obvious cause. Left and/or right ventricular systolic pump function of the heart is impaired, leading to progressive cardiac enlargement and finally heart failure. These processes are recently described by the term "cardiac remodeling". On the level of the myocardial tissue the loss of myocytes (necrosis), the degradation of sarcomeric structures with an induction of the fetal gene program in parallel, the atrophy of cardiomyocytes as well as compensating hypertrophic myocytes in an environment of massive fibrosis with infiltrating cells are evident (Schaper *et al.* 1995(1); Schaper *et al.* 1995(2); Schaper *et al.* 1991; Sharma *et al.* 2004).

In accordance with the present invention the terms "myocarditis" and "inflammatory cardiomyopathy" both refer to a form of cardiomyopathy which results in the phenotype of dilated cardiomyopathy.

"Myocarditis" is characterized by an inflammatory cellular infiltrate in the heart. Since DCM and myocarditis are regarded to be closely linked it is hypothesised that myocarditis is one face of DCM while the phases of myocarditis might be different. Phase 1 is dominated by viral infection itself, phase 2 by the onset of most likely multiple autoimmune reactions, and phase 3 by the progression to cardiac dilatation without an infectious agent and cardiac inflammation (Mason 2003). "Inflammatory cardiomyopathy" is defined as myocarditis in association with cardiac dysfunction (Maisch *et al.* 2005).

"Ischemic cardiomyopathy", in accordance with the present invention, refers to a weakness in the muscle of the heart due to inadequate oxygen delivery to the myocardium. The most common causes of ischemic cardiomyopathy are coronary artery diseases. Typically, the area of the heart affected by a myocardial infarction will initially become necrotic and will then be replaced by scar tissue (fibrosis). This fibrotic tissue is akinetic, i.e. it is no longer muscle and cannot contribute to the heart's function as a pump. If the akinetic region of the heart is substantial enough, the affected side of the heart (i.e. the left or right side) will go into failure. This failure is the functional result of an ischemic cardiomyopathy.
All of the diseases described herein are well known to the skilled person and are defined in accordance with the prior art and the common general knowledge of the skilled person.

### The figures show:

**Figure 1****: Increased numbers of macrophages correlate with the level of FGP in DCM patients.** Immunostaining with CD68 (E) marks macrophages in controls (CON) and patients with dilative cardiomyopathy (DCM;). Atrial natriuretic petide (ANP; A, F) is increased in cardiomyocytes of DCM patients. Note some unspecific signals caused by lipofuscin (pink). Western blots show dedifferentiation by increases of α-smooth muscle actin (B, α-SM-actin), the 95kDa B-Raf isoform (C), and destrin (D). Immunofluorescence shows the presence of destrin in the perinuclear region of DCM patients (D). In contrast, in normal human myocardium destrin is almost completely absent in this region. Destrin localizes also to the intercalated disc (D, arrows in yellow border frame). Pan-actin, actinin and troponin served as loading controls. For comparison patients with mild aortic valvular stenosis (AS) are included in the blots. Patient history is documented for AS and DCM in reference (Group 1 (Hein *et al*. 2003) and (Heling *et al.* 2000), respectively. Statistical analysis is shown in the corresponding graphs.

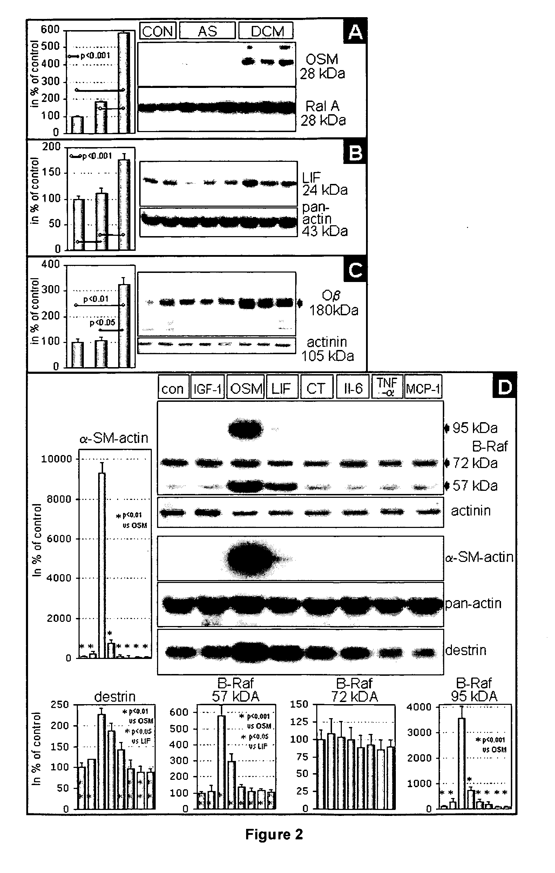

**Figure 2****: Oncostatin M is increased in DCM patients and induces more potently FGP than other members of the interleukin-6 class.** Western blots show increased levels of oncostatin M (OSM; A), its receptor (OSMRβ; C) and leukemia inhibitory factor (LIF, B) in patients with dilated cardiomyopathy (DCM). Patient history is the same as in figure 1. (D) Western blots demonstrate that OSM induces more potently FGP than LIF, cardiotrophin (CT), interleukin-6 (II-6), tumor necrosis factor-α (TNF-α) and monocyte chemotactic protein-1 (MCP-1) after 10d of serum-free culture. Parameters of FGP were the 95 kDa B-Raf isoform, α-smooth muscle actin (α-SM-actin) and destrin. Statistical analysis is shown in the corresponding graphs.
**Figure 3****: Inhibition of the MEK/ERK pathway abolishes oncostatin M induced FGP and** remodelling of cardiomyocytes. (A) Phase contrast micrographs of 8 day old serum-free cultures demonstrate that OSM induces more potently myocyte lengthening than LIF and that UO abolishes this effect. (B) Imunostaining for α-smooth muscle actin (α-SM-actin, red) and myomesin (green) a marker of mature sarcomeres. The presence of 2% serum did not inhibit sarcomeric loss and α-SM-actin accumulation in 12d old OSM-treated cardiomyocytes. For comparison untreated control (con) and insulin-like growth factor-1 (IGF-1) are depicted. Western blots demonstrate that UO126 (UO) abolished the activation of Erk1/2 (C) after 10 minutes and FGP after 10 days visible in the complete inhibition of the reexpression of the 95 kDa B-Raf isoform, α-smooth muscle actin (α-SM-actin), destrin (D) and loss of secreted atrial natriuretic peptide (ANP, E). Note in order to demonstrate equal loading the Western Blot of ANP is integrated into the ponceau stained membrane which is converted into grey. Statistical analysis is shown in the corresponding graphs.

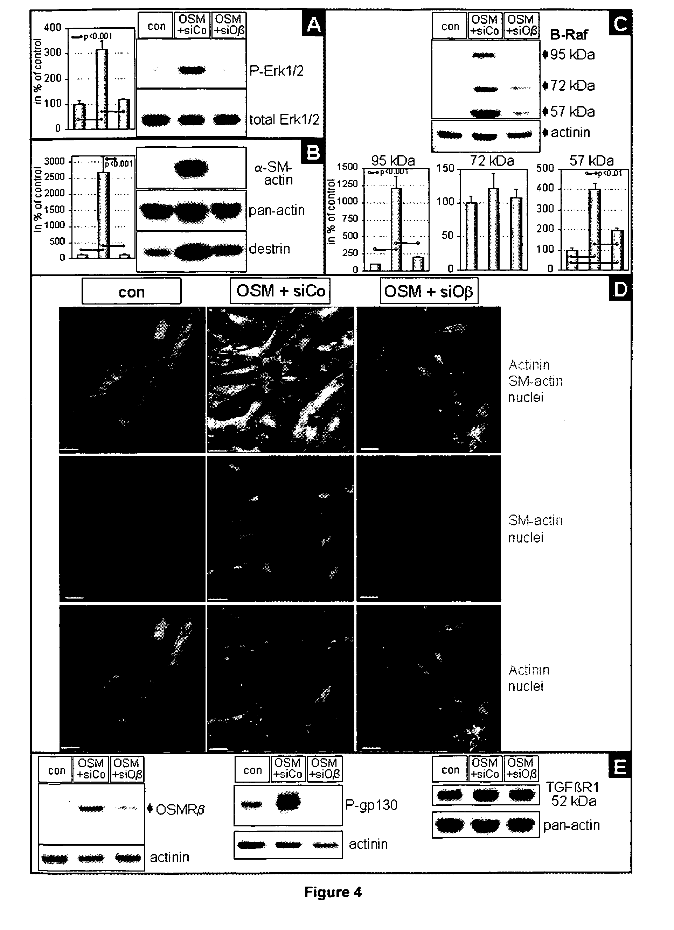

**Figure 4****: Knock down of the oncostatin M receptor β abolishes oncostatin M induced Erk1/2** activation, FGP and remodelling of cardiomyocytes. Cardiomyoctes were pretreated for 3 days with siRNA targeting the OSMRβ. Western blots demonstrate that OSMβ knock down abolished the activation of Erk1/2 (A) after 10 minutes and FGP after 10 days visible in the inhibition of the reexpression of the 95 kDa B-Raf isoform (C), α-smooth muscle actin (α-SM-actin) (B), destrin (B) and the loss of secreted atrial natriuretic peptide (ANP, Fig.3 E). Immunofluorescent pictures demonstrate that the knock down of the OSMRβ did not only lead to a downregulation of α-SM-actin but also to an almost complete inhibition of cell growth (D) of 10 days old OSM treated cultures. (E) siRNA suppressed the OSM-induced upregulation of the OSMRβ as well as the phosphorylation of the coreceptor gp130 but did not affect other receptors such as transforming growth factor-β receptor 1 (TGFβR1) after 10 days. Statistical analysis is shown in the corresponding graphs.
**Figure 5****: Oncostatin M activates the gp130/Raf/Erk axis and induces FGP in cardiac tissue.** 6 months old mice have been treated with oncostatin M (OSM) as described in the method section for 14 days. Western blots of OSMRβ (A) P-gp130 (B), P-Erk1/2 (C), α-smooth muscle actin (G) α-SM-actin), destrin (H), B-Raf isoforms (E) and atrial natriuretic peptide (D; ANP) demonstrate that OSM induces in the cardiac ventricle FGP and remodeling comparable to OSM treated cultured cardiomyocytes (Fig.2). Statistical analysis is shown in the corresponding graphs. Immunofluorescent pictures of OSM treated animals show increases of cytoplasmic α-smooth muscle actin ((G), α-SM-actin), ANP positive cardiomyocytes (D) and increases in destrin at the area of the intercalated disc (H) versus control (CON). The green (yellow) arrow in CON (OSM) marks an artery (G (H)). Nuclei are blue. Note: For B-Raf multiple bands are potentially arising from modifications and/or alternative splicing. (E) The level of LIF did not change (F).
**Figure 6****: Protein levels of oncostatin M and its receptor β correlate with the level of FGP in transgenic mice with cardiac restricted overexpression of monocyte chemotactic protein-1.** 6 months old mice with cardiac restricted overexpression of monocyte chemotactic protein-1 (MCP-1) show elevated numbers of invading cells into the cardiac tissue, which are mainly macrophages as evidenced by CD11b immunostaining (E). In controls and OSM treated animals few cells were positive (E). Western blots of cardiac tissue demonstrate increased levels of OSMRβ (A), P-gp130 (C), OSM (B), all B-Raf isoforms (D), α-SM-actin ((F), α-smooth muscle actin), destrin (G) and atrial natriuretic peptide (H; ANP) in MCP-1 animals in comparison to age-matched wild-type mice (WT).. Immunofluorescent pictures show massive increases in α-SM-actin (F) and ANP (H) positive cardiomyocytes. Wild-type animals correspond to the controls in figure 5. Destrin is increased throughout the cell (G, circle) in MCP-1 transgenes. Statistical analysis is shown in the corresponding graphs.
**Figure 7****: Inactivation of the oncostatin M receptor β by specific antibodies reduces FGP and** improves the ejection fraction in monocyte chemotactic protein-1 transgenic mice. 4 months old mice with cardiac restricted overexpression of monocyte chemotactic protein-1 (MCP-1) were treated by intravenous tail injection with 50µg of an oncostatin M receptor neutralizing antibody (AbOβ) or 50µg of isotype matching antibody (AbCon) as described in the method section. Data obtained with AbOβ are shown in dark green columns and that obtained with AbCon in light green columns. Statistical analysis is shown in the corresponding graphs with significance marked by (*). P-values less than 0.05 were considered statistically significant. (A) Western Blot analysis of 6 months old mice reveals a significant 35-50% reduction in the level of oncostatin M receptor (Oβ), phospho-gp130, the 95kDa B-Raf isoform, destrin and ANP. Pan-actin, pan-actinin and Ral A served as loading controls. (B) Specifity and neutralizing activity of about 60% of the AbOβ is demonstrated in oncostatin M stimulated cultured cardiomyocytes. (C) Magnetic resonance imaging reveals an increase in end-diastolic left ventricular mass and end-systolic volume in both groups. However, the left ventricular ejection fraction of transgenes treated with the AbOβ was significantly improved in comparison to the isotype treated animals indicating an improved contractile performance. (D) Model of the role of the oncostatin M receptor in heart failure. An initial injury leads to the invasion of macrophages which release oncostatin M (OSM). Activation of the OSM receptor exerts protective effects on cardiomyocytes through induction of FGP and reduces inflammation by downregulation of macrophage invasion and through the secretion of anti-inflammatory molecules. The chronic activation of the receptor contributes to the loss of cardiac function and finally heart failure.

The examples illustrate the invention.

### Example 1: Materials and methods

**Materials.** Basic medium consisting of medium 199 with Earle's balanced salts was supplemented as previously described (Kubin *et al.* 1999). UO126 and antibodies directed against phospho-Erk1/2, pan-actin and transforming growth factor-β receptor 1 were from Cell Signalling Technology. Antibodies recognizing, Ral A, B-Raf, Erk1/2 and phospho-gp130 were from Becton Dickinson. Note that the 67 kDa band of B-Raf runs under the Western Blot conditions presented here as 57 kDa band. Antibodies against troponin, destrin, α-smooth muscle actin and α-actinin were from Sigma. Antibodies recognizing oncostatin M receptor β were purchased from ProteinTech Group and R&D systems. Antibodies against atrial natriuretic peptide were from Chemicon and R&D systems. Antibodies recognizing leukaemia inhibitory factor were from R&D systems. All recombinant mouse proteins were from CellConcepts with the exception of mouse oncostatin M and leukemia inhibitory factor which were obtained from Sigma. Oncostatin M receptor beta siRNA (ON-TARGETplusSMARTpool L-087727-01-0010, NM_001005384) with the corresponding transfection kit was obtained from Dharmacon and cardiomyocytes were transfected according to the manufacturer's protocol. A polyclonal rabbit antibody directed against the sequence H2N-SAGPGPNETFTKVTTC-CONH2 of the mouse oncostatin M receptor was obtained from Eurogentec.

**MCP-1 transgenic animals, intraperitoneally injection of oncostatin M and culture of adult** cardiomyocytes. The generation of transgenic mice (from FVB/N mice) with cardiac restricted overexpression of monocyte chemotactic protein-1 has been previously described (Kolattukudy *et al.* 1998). 6 months old FVB mice were injected intraperitoneally twice daily with a volume of 0.2 ml of phosphate buffered saline containing 5% fetal calf serum without (CON) or with 60 ng oncostatin M (OSM) per gram of animal for fourteen days. Ventricular cardiac myocytes of 6-mo-old rats were isolated as previously described (Kubin *et al.* 1999). Myocytes were plated at a density of 1.5x10⁴ cells/cm² on laminin-coated (10 µg/ml; Sigma) chamber slides or 6 well dishes (Nunc) in basic medium. Myocytes were allowed to recover for 2 days in 2% fetal calf serum (FCS). Thereafter, basic medium, FCS, 50 ng/ml insulin-like growth factor-1 or 20 ng/ml of cytokines were added as indicated. Media were replaced every other day. UO126 (Cell Signaling Technology) was added 1h prior to stimulation. All experiments were performed in duplicate and constantly treated with 10 µM 1-(β-D-arabinofuranosyl)cytosine to prevent nonmyocyte growth. Determination of secreted ANP by Western Blot was performed after ultrafiltration (Millipore).

**MRI procedure and injection of oncostatin M receptor neutralizing antibodies into the tail vein.** Cardiac magnetic resonance imaging was performed as previously described documented (Ziebart *et al*. 2008). 50µg of oncostatin M receptor neutralizing antibody diluted in PBS (50µl total volume) were injected into the tail vein of four months old mice 5 times in a three day interval. Isotype matching antibodies served as control.

**Human heart tissue.** We obtained hearts from patients at the time of transplantation surgery. As previously documented (Heling *et al.* 2000), patients with end-stage heart failure (ejection fraction ≤ 20%;) due to dilative cardiomyopathy (DCM) were studied. Hearts from donors with normal left ventricular function not used for transplantation served as controls. As previously documented (Hein *et al*. 2003) (group1), left ventricle myectomies were obtained from patients with mild valvular aortic stenosis undergoing aortic valve replacement.

**Preparation of protein extracts and Western Blot analysis.** Tissue samples were sonicated in lysis buffer (0.1M Tris-HCl, 10% sodium dodecylsulfate (SDS), 10mM ethylenediamine tetraacetic acid, 40mM dithiothreitol (DTT); pH 8.0) containing inhibitors as previously described (Vogel *et al.* 2006). Cultures were washed twice with PBS and harvested by scraping in lysis buffer. After heating for 1 minute at 99°C debris was removed by centrifugation for 2 min at 14000g. Twenty micrograms of protein extracts were separated on 4%-12% SDS-PAGE gradient gels and processed as previously described (Vogel *et al.* 2006). Immunoreactive proteins were visualized on the VersaDoc (BioRad) and analyzed using the QuantityOne software (BioRad).

**Phase-contrast micrographs, immunocytochemistry and fluorescence microscopy.** Phase-contrast micrographs were taken with a 20x lens. For immunocytochemistry and fluorescence microscopy samples were processed as previously described (Kubin *et al.* 1999).

**Statistics.** For statistical analysis following tests (two-tailed) were used: a) three or more groups: 1 factorial anova and subsequent Bonferroni multiple comparisons respectively Kruskal-Wallis test and subsequent Dunn's multiple comparisons in the case of missing normality and/or heterogeneous variance of the data b) two groups: unpaired t-test respectively Mann-Whitney-U-test in the case of missing normality and/or heterogeneous variance of the data. P-values less than 0.05 were considered statistically significant.

### Example 2: DCM patients show FGP

Confocal micrographs demonstrated increased numbers of macrophages in the interstitial space of DCM patients (Fig. 1E). These increases correlate with the amount of atrial natriuretic peptide (ANP) in myocytes (Fig. 1A, F). ANP, a regulator of diuresis and a vasodilator, is produced in the mammalian embryo by myocytes of atria and ventricles. In the normal adult heart, the production of ANP is mainly restricted to the atria and its re-expression in the ventricle is coupled to hemodynamically overloaded and failing hearts (Kaganovsky *et al.* 2001; Poulos *et al.* 1996). Since the re-expression of ANP is linked to the fetal gene program (FGP), we further analysed dedifferentiation markers. α-SM actin decreases from birth to adulthood in myocardial tissue and is present in the adult heart mainly in smooth muscle cells. In addition, the protein level of the 95 kDa isoform B-Raf declines in rat heart from birth to adulthood and is not detected in isolated adult rat cardiomyocytes (Kim *et al*. 1998). Since reorganisation of myocyte structure involves depolymerization and polymerization of actin, we also analyzed the actin depolymerizing protein destrin by immunostaining and western blotting. Destrin is barely detectable in normal human myocardium (Fig. 1D). In contrast, in diseased myocardium destrin is increased in the perinuclear region of the cardiomyocyte (Fig. 1 D) and localizes to the intercalated disc (Fig. 1D, arrows in yellow border frame). Furthermore, Western Blots demonstrate increases in α-smooth muscle actin (Fig. 1B), the 95 kDa isoform of B-Raf (Fig. 1C) in DCM patients. For comparison patients with isolated aortic valvular stenosis (AS) of an ejection fraction ≤ 60% were analysed and are documented in detail as group 1 in reference (Hein *et al.* 2003).

### Example 3: DCM patients show increased levels of OSM and its receptor β

We investigated whether aspects of the syndrome of heart failure can be explained by direct effects of cardioactive cytokines on cardiomyocytes. Since conditioned media obtained from macrophages exerted potent activities on the differentiation status and morphology of cultured adult cardiomyocytes (not shown), we asked whether morphogens regarded to be restricted to macrophages might occur in increased amounts in failing hearts. Western blot analysis demonstrates that the levels of OSM (Fig. 2A) and its receptor β (Fig. 2C) in DCM patients correlate with the degree of FGP (Fig. 1). We also confirmed increases in the protein amount of LIF (Fig. 2B) which has been reported on the mRNA level elsewhere (Eiken *et al.* 2001).

### Example 4: OSM is the most potent cytokine of the interleukin-6 class of cytokines

We tested the effects of the interleukin-6 class in their potency on cardiomyocytes since these cytokines and the common receptor gp130 are extensively described in the context of hypertrophy, cell survival and heart failure (Fischer and Hilfiker-Kleiner 2007). LIF, cardiotrophin and II-6 showed only a limited capacity to induce dedifferentiation as evidenced by the re-expression of the 95kDa B-Raf, α-SM actin and destrin (Fig. 2D). An increasing body of clinical and experimental evidence suggests that tumour necrosis factor-α (TNF-α) (Seta *et al.* 1996) and MCP-1 (Kolattukudy *et al.* 1998) play a pathogenic role in failing hearts. However, neither TNF-α nor MCP-1 induced FGP (Fig. 2D). One of the most striking effects of OSM on cardiomyocytes is the re-expression of the 95 kDa B-Raf isoform. B-Raf is regarded as the main effector of GTP-bound Ras signaling and considered as the dominating entry point to ERK signalling. Rafs are involved in cell growth and differentiation through the regulation of transcription and cytoskeletal rearrangements. This opens up the question whether ERKs mediate FGP and remodelling.

### Example 5: OSM induces FGP utilizing the MEK/Erk module

In the absence of serum OSM induced within 8 days remodelling of cardiomyocytes primarily visible in cell lengthening and was more pronounced than in LIF-stimulated cells. The MEK1/2 inhibitor UO126 completely abolished OSM-induced lengthening (Fig. 3A). In contrast, in the presence of 2% serum, OSM treatment lead to an enlargement of the surface area in addition to lengthening after 12 days. A massive loss of sarcomeres is visible in OSM-stimulated cultures in comparison to 2% serum treated controls (Fig. 3B), indicating that OSM induces sarcomeric degradation. This fits with the loss of sarcomeric structure in failing cardiomyocytes as documented earlier (Hein *et al*. 2003; Heling *et al.* 2000; Schaper *et al.* 1995(1); Schaper *et al.* 1995(2)). IGF-1, a well-known stimulator of sarcomerogenesis, served as positive control (Fig. 3B). Dedifferentiation and remodelling of myocytes is visible in the massive re-expression of α-SM actin throughout the myocyte (Fig. 3B). The inhibition of cell lengthening by UO126 correlated with the inhibition of Erk1/2 activation after 10 minutes (Fig. 3C). Activation of this pathway appears to be a prerequisite for FGP as evidenced by the loss of re-expression of α-SM actin, the 95 kDa B-Raf and destrin (Fig. 3D) and the reduced secretion of ANP (Fig. 3E) in the presence of the inhibitor after 10 days. We then asked whether the OSMRβ is the crucial regulator of FGP and remodelling.

### Example 6: Knock down of the oncostatin M receptor β by siRNA abolishes OSM activities

Before starting experiments, cardiomyocytes were pretreated for 3 days with siRNA targeting the OSM receptor β in the presence of 2% serum. Knock down of OSMRβ abolished Erk1/2 activation after 10 minutes (Fig. 4A). The degree of inhibition of dedifferentiation after 10 days was comparable to that observed with UO126 visible in the reduction of the 95 kDa B-Raf isoform (Fig. 4C), α-SM actin and destrin (Fig. 4B) and the abolished secretion of ANP (Fig. 3E). Immunostaining demonstrates that the loss of OSMRβ did not only lead to a down-regulation of dedifferentiation but also to an almost complete inhibition of cell growth (Fig. 4D). siRNA suppressed the OSM-induced up-regulation of Oβ as well as the phosphorylation of the coreceptor gp130 (Fig. 4E) but did not affect other receptors such as transforming growth factor receptor β1. Based on the "cytokine hypothesis" (Seta *et al.* 1996), we stated that an adequate concentration of OSM is sufficient to induce FGP in mice.

### Example 7: OSM activates the gp130/Raf/MEK/Erk axis and induces FGP in cardiac tissue

Six months old mice underwent an OSM-treatment schedule as described in the method section following a regimen described for LIF (Metcalf *et al.* 1990). We observed significant increases in the amount of OSMRβ (Fig. 5A) and in the phosphorylation level of the serine residue 782 of gp130 (Fig. 5B). This site is regarded as a major site for gp130 phosphorylation (Gibson *et al.* 2000). Moderate increases in P-Erk1/2 reflect the short living nature of this kinase pathway (Fig. 5C) consistent with our observation that OSM induced a rapid (Fig. 3C) but transient induction of ERK phosphorylation in cell culture (not shown). In addition, increased amounts of all B-Raf isoforms indicate an important role of this kinase pathway in FGP and remodelling (Fig. 5E). It has to be noted that more than three B-Raf bands were detectable in the range between 57 kDa and 115 kDa in OSM treated animals, which confirms the existence of multiple bands of B-Raf in mice tissues (Barnier *et al.* 1995). Modifications, such as phosphorylations known to regulate Raf activities, might contribute to the complexity of the detected bands in addition to alternative splicing. Consistent with our cell culture data, OSM-treatment lead to an increased level of α-SM actin (Fig. 5G), destrin (Fig. 5H) and ANP (Fig. 5D) in myocardial tissue. Immunostaining demonstrated that α-smooth muscle actin is confined in normal mouse myocardium to smooth muscle cells but is re-expressed in cardiomyocytes after OSM injection (Fig. 5G). OSM-treatment lead to an increase of destrin in the myocardial tissue which is mainly confined to the area of the intercalated disc (Fig. 5H). No changes in LIF were detected (Fig. 5F). To confirm our hypothesis that invading monocytes induce FGP by OSM secretion, we analysed a transgenic mouse strain with restricted overexpression of monocyte chemotactic protein-1 in the cardiac tissue.

Since OSMRβ lacks intrinsic tyrosine kinase activity, ligand binding activates the Janus tyrosine kinases (JAK) which results in rapid phosphorylation of the tyrosine residues on the cytoplasmic tail of both receptor units. These phosphorylation sites serve as docking sites for the STATs which are then phosporylated by JAKs. STATs dissociate and translocate to the nucleus where they regulate gene transcription. In addition to the JAK-STAT pathway, OSM employs the Ras-Raf-MEK-Erk module. Raf is considered as the main effector of GTP-bound Ras signaling, and Rafs are involved in cell growth and differentiation through the regulation of transcription and cytoskeletal rearrangements. The regulation of Raf is, however, complex since B-Raf and not the abundant C-Raf (Raf-1) is regarded as the major activator of MEK1/2. B-Raf has strongly elevated basal kinase activity and requires only Ras-mediated membrane recruitment for activation. By contrast, C-Raf and A-Raf require further inputs from tyrosine and serine kinases (Wellbrock *et al.* 2004). Thus, the re-expression of the 95kDa B-Raf isoform after OSM treatment underlines the importance of this pathway in dedifferentiating cardiomyocytes. Raf phosphorylates and activates MEK1/2 that in turn phosphorylate and activate Erk1/2. The importance of the OSMRβ/MEK/Erk axis as a central regulator of the fetal gene program was visualized by targeting components of this axis. The phosphorylation of Erk1/2 was abolished through the specific MEK inhibitor UO126 as well as through siRNA targeting the OSMRβ. The degree of inhibition of remodelling and dedifferentiation was almost identical in UO126 and siRNA treated cardiomyocytes documented through the abolishment of cell lengthening, the reexpression of ANP, α-SM actin and the 95kDa B-Raf isoform.

### Example 8: Overexpression of monocyte chemotactic protein-1 in cardiac tissue leads to invasion of macrophages, increases in OSM and FGP

Six months old MCP-1 transgenic mice show increased numbers of infiltrating cells, mainly macrophages as evidenced by CD11b immunostaining (Fig. 6E). This age is associated with ventricular dilatation and heart failure (Kolattukudy *et al.* 1998). Transgenic mice show an increased level of OSMRβ (Fig. 6A) and an elevated phosphorylation level at serine residue 782 of its coreceptor gp130 (Fig. 6C). In addition, increases in the ligand OSM, but not LIF (Fig. 6B), indicate an OSMRβ mediated signalling. This is reflected in the activation of FGP similar to that observed in mice treated with OSM (Fig. 5). The levels of α-SM actin (Fig. 6F) and ANP (Fig. 6H) are dramatically elevated in transgenic mice in comparison to age-matched wild type mice. Immunostaining demonstrates that α-SM actin (Fig. 6F) and ANP are re-expressed in cardiomyocytes (Fig.6H). All B-Raf isoforms are elevated (Fig. 6D). Destrin appears increased to the same extent as in OSM injected animals (Fig. 5G) and is distributed throughout the cell (Circle, Fig. 6G). Confocal images of age-matched wild-type mice are shown in figure 5. To further investigate our hypothesis that the OSMRβ receptor mediated signal transduction is responsible for the FGP in these transgenic mice, we tested whether the blockade of OSMRβ reverses FGP.

### Example 9: Inactivation of the oncostatin M receptor β by specific antibodies reduces FGP and improves the ejection fraction in monocyte chemotactic protein-1 transgenic mice

Magnetic resonance imaging (MRI) of four months and six months old transgenic mice was performed as previously described (Ziebart *et al.* 2008). 4 months old mice received an injection into the tail vein of 50µg oncostatin M receptor neutralizing antibodies or isotype matching antibodies 5 times in a three day interval. We observed significant reductions in the amount of OSMRβ, in the phosphorylation level of gp130, in the amount of the 95kDa B-Raf and in the level of destrin and ANP (Fig. 7A) in animals treated with Oβ blocking antibodies. This antibody reduced the level of gp130 phosphorylation by about 60% (Fig. 7B) after stimulation of cardiomyocytes with OSM. We did not see any differences between the two groups in the amount of α-SM-actin which might be due to the fact that four months old transgenic mice already contain high amounts of α-SM-actin (not shown). Heart function and left ventricular dilatation were determined by MRI (Fig. 7C). All transgenic mice, independently of the treatment, showed a gross dilatation of the left ventricle, increases in the end-diastolic left ventricular mass and the end-systolic volume after six months in comparison to the data of the four months old animals. MRI revealed signs of global left ventricular dysfunction in both groups after 6 months. However, the left ventricular ejection fraction of transgenic mice treated with the OSMRβ neutralizing antibody was significantly improved in comparison to the isotype treated animals. This indicates that the blockade of OSMRß reduces the atrophy and FGP in cardiomyocytes thus preserving myocardial function. Figure 7D summarizes these data.

### References

Akira, S et al. Molecular cloning of APRF, a novel IFN-stimulated gene factor 3 p91-related transcription factor involved in the gp130-mediated signaling pathway. Cell 77(1):63-71 (1994).
Ausma, J. et al. Chronic Ischemic Viable Myocardium in Man: Aspects of Dedifferentiation. Cardiovascular Pathology 4, 29-37 (1995).
Ausma, J. et al. Dedifferentiated cardiomyocytes from chronic hibernating myocardium are ischemia-tolerant. Mol Cell Biochem 186, 159-68 (1998).
Barnier, J.V., Papin, C., Eychene, A., Lecoq, O. & Calothy, G. The mouse B-raf gene encodes multiple protein isoforms with tissue-specific expression. J Biol Chem 270, 23381-9 (1995).
Betz, U.A. et al. Postnatally induced inactivation of gp130 in mice results in neurological, cardiac, hematopoietic, immunological, hepatic, and pulmonary defects. J Exp Med 188, 1955-65 (1998).
Charest, DL et al. Molecular cloning, expression, and characterization of the human mitogen-activated protein kinase p44erk1. Mol Cell Biol. 13(8):4679-90 (1993).
Dispersyn, G.D. et al. Dissociation of cardiomyocyte apoptosis and dedifferentiation in infarct border zones. Eur Heart J 23, 849-57 (2002).
Ebelt, H. et al. Cellular cardiomyoplasty: improvement of left ventricular function correlates with the release of cardioactive cytokines. Stem Cells 25, 236-44 (2007).
Eiken, H.G. et al. Myocardial gene expression of leukaemia inhibitory factor, interleukin-6 and glycoprotein 130 in end-stage human heart failure. Eur J Clin Invest 31, 389-97 (2001).
Eppenberger-Eberhardt, M. et al. IGF-I and bFGF differentially influence atrial natriuretic factor expression and α-smooth muscle actin expression in cultured atrial compared to ventricular adult rat cardiomyocytes. J Mol Cell Cardiol 29, 2027-2039 (1997).
Fischer, P. & Hilfiker-Kleiner, D. Survival pathways in hypertrophy and heart failure: the gp130-STAT axis. Basic Res Cardiol 102, 393-411 (2007).
Gibson, R.M. et al. Phosphorylation of human gp130 at Ser-782 adjacent to the Di-leucine internalization motif. Effects on expression and signaling. J Biol Chem 275, 22574-82 (2000).
Goedert, M et al. Activation of the novel stress-activated protein kinase SAPK4 by cytokines and cellular stresses is mediated by SKK3 (MKK6); comparison of its substrate specificity with that of other SAP kinases. EMBO J. 16(12):3563-71 (1997).
Gwechenberger, M., Moertl, D., Pacher, R. & Huelsmann, M. Oncostatin-M in myocardial ischemia/reperfusion injury may regulate tissue repair. Croat Med J 45, 149-57 (2004).
Hein, S. et al. Progression from compensated hypertrophy to failure in the pressure-overloaded human heart: structural deterioration and compensatory mechanisms. Circulation 107, 984-91 (2003).
Heling, A. et al. Increased expression of cytoskeletal, linkage, and extracellular proteins in failing human myocardium. Circ Res 86, 846-53 (2000).
Hirota, H. et al. Loss of a gp130 cardiac muscle cell survival pathway is a critical event in the onset of heart failure during biomechanical stress. Cell 97, 189-98 (1999).
Hou, J et al. Identification and purification of human Stat proteins activated in response to interleukin-2. Immunity. 2(4):321-9 (1995).
Ichihara, M., Hara, T., Kim, H., Murate, T. & Miyajima, A. Oncostatin M and leukemia inhibitory factor do not use the same functional receptor in mice. Blood 90, 165-73 (1997).
Jiang, Y et al. Characterization of the structure and function of a new mitogen-activated protein kinase (p38beta). J Biol Chem. 271(30):17920-6 (1996).
Kaganovsky, E. et al. Occurrence and distribution of atrial natriuretic peptide-containing cells in the left ventricle of hypertensive rats. Effect of antihypertensive treatment. Cell Tissue Res 303, 57-67 (2001).
Kim, S.O., Irwin, P., Katz, S. & Pelech, S.L. Expression of mitogen-activated protein kinase pathways during postnatal development of rat heart. J Cell Biochem 71, 286-301 (1998).
Kolattukudy, P.E. et al. Myocarditis induced by targeted expression of the MCP-1 gene in murine cardiac muscle. Am J Pathol 152, 101-11 (1998).
Kubin, T. et al. Microvascular endothelial cells remodel cultured adult cardiomyocytes and increase their survival. Am J Physiol 276, H2179-87 (1999).
Lechner, C et al. ERK6, a mitogen-activated protein kinase involved in C2C12 myoblast differentiation. Proc Natl Acad Sci U S A. 93(9):4355-9 (1996).
Lee, JC et al. A protein kinase involved in the regulation of inflammatory cytokine biosynthesis. Nature 372(6508):739-46 (1994).
Lee, JD et al. Primary structure of BMK1: a new mammalian map kinase. Biochem Biophys Res Commun. 213(2):715-24 (1995).
Lin, JX et al. Cloning of human Stat5B. Reconstitution of interleukin-2-induced Stat5A and Stat5B DNA binding activity in COS-7 cells. J Biol Chem. 271(18):10738-44 (1996).
Malik, N et al. Molecular Cloning, Sequence Analysis, and Functional Expression of a Novel Growth regulator, Oncostatin M. Mol and Cell Biol Vol. 9, No. 7, pp2847-2853 (1989)
Martire, A. et al. Cardiac overexpression of monocyte chemoattractant protein-1 in transgenic mice mimics ischemic preconditioning through SAPK/JNK1/2 activation. Cardiovasc Res 57, 523-34 (2003).
Mason JW. Myocarditis and dilated cardiomyopathy: An inflammatory link. Cardiovasc Res 60, 5-10 (2003).
Metcalf, D., Nicola, N.A. & Gearing, D.P. Effects of injected leukemia inhibitory factor on hematopoietic and other tissues in mice. Blood 76, 50-6 (1990).
Minamoto, S et al.: Cloning and functional analysis of new members of STAT induced STAT inhibitor (SSI) family: SSI-2 and SSI-3. Biochem Biophys Res Commun. 237(1):79-83 (1997).
Mire-Sluis, A.R. & Thorpe, R. (eds.). Cytokines, (Academic Press, San Diego, 1998).
Mosley, B. et al. Dual Oncostatin M (OSM) Receptors. J Biol Chem 271, No. 50, pp32635-32643 (1996).
Owaki H et al. Extracellular signal-regulated kinases in T cells: characterization of human ERK1 and ERK2 cDNAs. Biochem Biophys Res Commun. 182(3):1416-22 (1992).
Packer, M. Evolution of the neurohormonal hypothesis to explain the progression of chronic heart failure. Eur Heart J 16 Suppl F, 4-6 (1995).
Poulos, J.E., Gower, W.R., Jr., Sullebarger, J.T., Fontanet, H.L. & Vesely, D.L. Congestive heart failure: increased cardiac and extracardiac atrial natriuretic peptide gene expression. Cardiovasc Res 32, 909-19 (1996).
Schaper, J. et al. Impairment of the myocardial ultrastructure and changes of the cytoskeleton in dilated cardiomyopathy. Circulation 83, 504-14 (1991).
Schaper, J., Hein, S., Scholz, D. & Mollnau, H. Multifaceted morphological alterations are present in the failing human heart. J Mol Cell Cardiol 27, 857-61 (1995).
Schaper, J. et al. Interactions between cardiomyocytes and extracellular matrix in the failing human heart. Z Kardiol 84 Suppl 4, 33-8 (1995).
Seta, Y., Shan, K., Bozkurt, B., Oral, H. & Mann, D.L. Basic mechanisms in heart failure: the cytokine hypothesis. J Card Fail 2, 243-9 (1996).
Schindler, C et al. Proteins of transcription factor ISGF-3: one gene encodes the 91-and 84-kDa ISGF-3 proteins that are activated by interferon alpha. Proc Natl Acad Sci U S A. 89(16):7836-9 (1992). Sharma, U.C. et al. Galectin-3 marks activated macrophages in failure-prone hypertrophied hearts and contributes to cardiac dysfunction. Circulation 110, 3121-8 (2004).
Seger, R et al. Human T-cell mitogen-activated protein kinase kinases are related to yeast signal transduction kinases. J Biol Chem. 267(36):25628-31 (1992).
Vasse, M. et al. Oncostatin M induces angiogenesis in vitro and in vivo. Arterioscler Thromb Vasc Biol 19, 1835-42 (1999).
Vogel, S. et al. MEK hyperphosphorylation coincides with cell cycle shut down of cultured smooth muscle cells. J Cell Physiol 206, 25-34 (2006).
Wang. Mitogen-Activated Protein Kinases in Heart Development and Diseases. Circulation 116: 1413-1423 (2007).
Wellbrock, C., Karasarides, M. & Marais, R. The RAF proteins take centre stage. Nat Rev Mol Cell Biol 5, 875-85 (2004).
Wilks, AF et al. Two novel protein-tyrosine kinases, each with a second phosphotransferase-related catalytic domain, define a new class of protein kinase. Mol Cell Biol. 11 (4):2057-65 (1991).
Zheng, CF et al. Cloning and characterization of two distinct human extracellular signal-regulated kinase activator kinases, MEK1 and MEK2. J Biol Chem. 268(15):11435-9 (1993).
Zarling, J.M. et al. Oncostatin M: A Growth Regulator Produced by Differentiated Histiocytic Lymphoma Cells. PNAS 83, 9739-9743 (1986).
Ziebart et al. Sustained persistence of transplanted proangiogenic cells contributes to neovascularization and cardiac function after ischemia. Circ Res. 2008 Nov 21; 103(11):1327-34.

## Claims

1. An inhibitor of the oncostatin M receptor ß or an inhibitor of an activator of the oncostatin M receptor ß for use in the treatment and/or prevention of heart failure.

2. A method of treating and/or preventing heart failure comprising administering a pharmaceutically effective amount of an inhibitor of the oncostatin M receptor ß or an inhibitor of an activator of the oncostatin M receptor ß to a subject in need thereof.

3. The inhibitor of claim 1 or the method of claim 2, wherein the inhibitor is an antibody or a fragment or derivative thereof, an aptamer, an siRNA, an shRNA, a miRNA, a ribozyme, an antisense nucleic acid molecule, modified versions of these inhibitors or a small molecule.

4. The inhibitor of claim 1 or the method of claim 2, wherein the activator of the oncostatin M receptor ß is oncostatin M.

5. A method of identifying an inhibitor of the oncostatin M receptor ß or an inhibitor of an activator of the oncostatin M receptor ß suitable as a lead compound and/or as a medicament for the treatment and/or prevention of heart failure, comprising the steps of:
(a) determining the level of oncostatin M receptor ß protein or oncostatin M receptor ß transcript in a cell;
(b) contacting said cell or a cell of the same cell population with a test compound;
(c) determining the level of oncostatin M receptor ß protein or oncostatin M receptor ß transcript in said cell after contacting with the test compound; and
(d) comparing the level of oncostatin M receptor ß protein or oncostatin M receptor ß transcript determined in step (c) with the oncostatin M receptor ß protein or oncostatin M receptor ß transcript level determined in step (a), wherein a decrease of oncostatin M receptor ß protein or oncostatin M receptor ß transcript level in step (c) as compared to step (a) indicates that the test compound is an inhibitor of the oncostatin M receptor ß or an inhibitor of an activator of the oncostatin M receptor ß suitable as a lead compound and/or as a medicament for the treatment and/or prevention of heart failure.

6. A method of identifying an inhibitor of the oncostatin M receptor ß or an inhibitor of an activator of the oncostatin M receptor ß suitable as a lead compound and/or as a medicament for the treatment and/or prevention of heart failure, comprising the steps of:
(a) determining the level of activity of an oncostatin M receptor ß target molecule in a cell containing oncostatin M receptor ß protein;
(b) contacting said cell or a cell of the same cell population with a test compound;
(c) determining the level of activity of the oncostatin M receptor ß receptor target molecule in said cell after contacting with the test compound; and
(d) comparing the level of activity of the oncostatin M receptor ß target molecule determined in step (c) with the level of activity of the oncostatin M receptor ß target molecule determined in step (a), wherein a decreased activity of the target molecule in step (c) as compared to step (a) indicates that the test compound is an inhibitor of the oncostatin M receptor ß or an inhibitor of an activator of the oncostatin M receptor ß suitable as a lead compound and/or as a medicament for the treatment and/or prevention of heart failure.

7. The method of claim 6, wherein the oncostatin M receptor ß target molecule is selected from JAK1, STAT1, STAT3, STATS, MEK1/2, ERK1/2, SOCS3, p38.

8. The method of any one of claims 5 to 7, wherein said cell comprising the oncostatin M receptor
ß is a cardiomyocyte.

9. The method of any one of claims 5 to 8, further comprising optimising the pharmacological properties of an inhibitor of the oncostatin M receptor ß or an inhibitor of an activator of the oncostatin M receptor ß identified as lead compound.

10. The method of claim 9, wherein the optimisation comprises modifying the inhibitor of the oncostatin M receptor ß or the inhibitor of an activator of the oncostatin M receptor ß to achieve:
i) modified spectrum of activity, organ specificity, and/or
ii) improved potency, and/or
iii) decreased toxicity (improved therapeutic index), and/or
iv) decreased side effects, and/or
v) modified onset of therapeutic action, duration of effect, and/or
vi) modified pharmacokinetic parameters (resorption, distribution, metabolism and excretion), and/or
vii) modified physico-chemical parameters (solubility, hygroscopicity, color, taste, odor, stability, state), and/or
viii) improved general specificity, organ/tissue specificity, and/or
ix) optimised application form and route
by
(a) esterification of carboxyl groups, or
(b) esterification of hydroxyl groups with carboxylic acids, or
(c) esterification of hydroxyl groups to, e.g. phosphates, pyrophosphates or sulfates or hemi-succinates, or
(d) formation of pharmaceutically acceptable salts, or
(e) formation of pharmaceutically acceptable complexes, or
(f) synthesis of pharmacologically active polymers, or
(g) introduction of hydrophilic moieties, or
(h) introduction/exchange of substituents on aromates or side chains, change of substituent pattern, or
(i) modification by introduction of isosteric or bioisosteric moieties, or
(j) synthesis of homologous compounds, or
(k) introduction of branched side chains, or
(I) conversion of alkyl substituents to cyclic analogues, or
(m) derivatisation of hydroxyl groups to ketales, acetales, or
(n) N-acetylation to amides, phenylcarbamates, or
(o) synthesis of Mannich bases, imines, or
(p) transformation of ketones or aldehydes to Schiffs bases, oximes, acetales, ketales,
enolesters, oxazolidines, thiazolidines
or combinations thereof.

11. The inhibitor of any one of claims 1 or 3 and 4, or the method of any one of claims 5 to 10, wherein the heart failure is selected from dilated cardiomyopathy, myocarditis, inflammatory cardiomyopathy and ischemic cardiomyopathy.
